# EUROPEAN PATENT APPLICATION

(11) **EP 2 929 785 A1**
(43) Date of publication of application: **14.10.2015**
(21) Application number: 14164082.1
(22) Date of filing: 09.04.2014
(51) Int. Cl.: A23J 7/00, A61K 8/55, A61K 47/48, A61K 31/00, A61K 31/575, A61K 31/661, A61K 31/6615, A61P 3/04, A61P 3/06

(54) **A PLA inhibitor for inhibition of fat absorption**

(71) Applicant: PAT GmbH, 81375 München (DE)
(72) Inventor: Stremmel, Wolfgang, 69120 Heidelberg (DE)
(74) Representative: Patent- und Rechtsanwälte Ullrich & Naumann

(57) **Abstract**

The present invention relates to the field of medical or cosmetic weight management. In particular, the present invention provides compounds for inhibition of fat absorption. Said compounds are characterized as phospholipase A inhibitors. In particular, the PLA inhibitors of the invention can be lysophospholipid-conjugates. The compounds of the invention can be used for therapeutic and cosmetic body fat reduction in a subject. Therapeutic use of the inventive compounds can in particular include obesity treatment. The present invention also provides methods, compositions and kits for weight management.

SEQUENCE LISTING

<110>  PAT GmbH

<120>  A PLA INHIBITOR FOR INHIBITION OF FAT ABSORPTION

<130>  5692/A/1003-EP

<160>  20

<170>  PatentIn version 3.5

<210>  1
<211>  20
<212>  DNA
<213>  artificial

<220>
<223>  primer for iPLA2beta

<400>  1
atcctgatga atttgagcga                                                    20


<210>  2
<211>  21
<212>  DNA
<213>  artificial

<220>
<223>  primer for iPLA2beta

<400>  2
caagtagaag ttccttgaac g                                                  21


<210>  3
<211>  20
<212>  DNA
<213>  artificial

<220>
<223>  primer for CD36

<400>  3
tgtaacccag gacgctgagg                                                    20


<210>  4
<211>  20
<212>  DNA
<213>  artificial

<220>
<223>  primer for CD36

<400>  4
gaaggttcga agatggcacc                                                    20


<210>  5
<211>  18
<212>  DNA

<213>  artificial

<220>
<223>  primer for FABPPM

<400>  5
gagggtcgga gccagctt                                                    18


<210>  6
<211>  20
<212>  DNA
<213>  artificial

<220>
<223>  primer for FABPPM

<400>  6
gtttccccag gatggtttgg                                                  20


<210>  7
<211>  30
<212>  DNA
<213>  artificial

<220>
<223>  primer for caveolin-1

<400>  7
aacgttctca ctcgctctct gctcgctgcg                                       30


<210>  8
<211>  24
<212>  DNA
<213>  artificial

<220>
<223>  primer for caveolin-1

<400>  8
gtacacttgc ttctcgctca gcac                                             24


<210>  9
<211>  19
<212>  DNA
<213>  artificial

<220>
<223>  siRNA control

<400>  9
gaugggaccu ggccaguga                                                   19


<210>  10
<211>  19
<212>  DNA
<213>  artificial

<220>

<223>  siRNA control

<400>  10
ucacuggcca ggucccauc                                                    19


<210>  11
<211>  17
<212>  DNA
<213>  artificial

<220>
<223>  siRNA-iPLA2beta

<400>  11
cccguacacc caccuau                                                      17


<210>  12
<211>  19
<212>  DNA
<213>  artificial

<220>
<223>  siRNA-iPLA2beta

<400>  12
auaggugugg guguacggg                                                    19


<210>  13
<211>  19
<212>  DNA
<213>  artificial

<220>
<223>  siRNA- iPLA2beta

<400>  13
cugacuacgu cucaaguga                                                    19


<210>  14
<211>  19
<212>  DNA
<213>  artificial

<220>
<223>  siRNA- iPLA2beta

<400>  14
ucacuugaga cguagucag                                                    19


<210>  15
<211>  19
<212>  DNA
<213>  artificial

<220>
<223>  siRNA-CD36

<400>  15

caaagagguc cuuauacgu                                                    19

<210>  16
<211>  19
<212>  DNA
<213>  artificial

<220>
<223>  siRNA-CD36

<400>  16
acguauaagg accucuuug                                                    19

<210>  17
<211>  19
<212>  DNA
<213>  artificial

<220>
<223>  siRNA-FABPPM

<400>  17
gccuuuaaga gggacacca                                                    19

<210>  18
<211>  19
<212>  DNA
<213>  artificial

<220>
<223>  siRNA-FABPPM

<400>  18
uggugucccu cuuaaaggc                                                    19

<210>  19
<211>  19
<212>  DNA
<213>  artificial

<220>
<223>  siRNA-caveolin1

<400>  19
cuaaacaccu caacgauga                                                    19

<210>  20
<211>  19
<212>  DNA
<213>  artificial

<220>
<223>  siRNA-caveolin1

<400>  20
ucaucguuga gguguuuag                                                    19

## Description

### BACKGROUND

Obesity, most often defined as abnormal or excessive fat accumulation that may impair health, commonly caused by a combination of excessive food energy intake, lack of physical activity and genetic susceptibility, is widely recognised as the largest and fastest growing public health problem in the developed and developing world. Worldwide obesity has nearly doubled since 1980, and it has been estimated that, in 2005, some 400 million adults worldwide were obese, with a total of 1.6 billion being overweight. Obesity is associated with substantial increases in morbidity, premature mortality, impaired quality of life and large healthcare costs. It increases the likelihood of various diseases, particularly heart disease, type 2 diabetes, obstructive sleep apnea, certain types of cancer, osteoarthritis and non-alcoholic steatohepatitis and is considered one of the most serious public health problems of the 21 st century.

Surprisingly, however, treatment options remain quite limited (Rogers RJ Dis Mod & Mech. 2012; 5:621-626 for review). With the exception of bariatic surgery there is no effective therapeutic measure available to control overeating habits. However, due to concerns about perioperative mortality, surgical complications and the frequent need for reoperation, these procedures tend to be reserved for the morbidly obese. Lifestyle changes, on the other hand, in the form of dieting and/or exercise per se do not generally produce marked or sustainable weight loss whereas effective psychological therapies, such as cognitive behavioural therapy, cannot easily be delivered on a mass scale nd long-term results are disappointing.

Beside a mental training strategy to control calorie intake and physical exercise programs, an alternative strategy to surgery is to develop therapeutic agents that can reduce body weight by decreasing the consumption or absorption of food, and/or by increasing energy expenditure. The history of the pharmacotherapy of obesity dates back to the 1930s. Many drugs used to treat obesity in the past have been discontinued because of their potential to be abused and their toxicities risk of psychiatric disorders including depression and suicidal tendencies increased risk of major adverse cardiovascular events (a composite of non-fatal heart attack, non-fatal stroke, resuscitation after cardiac arrest, and cardiovascular death). In addition, approved long-term therapy for obesity has only limited efficacy.

Few anti-obesity agents are also sold at lower dosage over-the-counter to a growing percentage of the population having a desire to reduce their body weight without medical indication, but for aesthetic reasons. However, many anti-obesity agents remain reserved for the morbidly obese due to their adverse side-effects, which are not compensated by their beneficial effects for normal or slightly overweight.

Thus, there exists a need for the development of novel drugs for body fat and weight reduction and/or control with high efficacy and long-term safety.

### SUMMARY

Using the human hepatocyte-derived tumor cell line HepG2, the present inventors found that fatty acid influx is mediated by a heterotetrameric plasma membrane protein complex consisting of plasma membrane fatty acid-binding protein (FABP_{PM}), caveolin1, cluster of differentiation (CD) 36, and calcium-independent membrane phospholipase A₂ (iPLA₂β). Blocking iPLA₂β with the bile acid-phospholipid conjugate ursodeoxycholatelysophosphatidylethanolamide (UDCA-LPE) caused the dissociation of the complex, thereby inhibiting fatty acid influx, and suppressed the synthesis of all subunits through a reduction in lysophosphatidylcholine and corresponding depletion of phosphorylated c-Jun N-terminal kinase (p-JNK1). These findings were substantiated by an observed 56.5% decrease in fatty acid influx in hepatocytes derived from iPLA₂β knockout mice.

In these mice it was indeed shown that iPLA*2*β -/- hepatocytes have a reduced fatty acid influx capacity. Human studies have shown that a mutation in PNPLA3, a member of the iPLA2 family, conferred susceptibility toward NASH by mechanisms inhibiting release of fat from hepatocytes (Krarup et al. (2012), PLoS One 7, e40376). The human homolog of the group VIA PLA2 (iPLA2β) examined here is encoded by PNPLA9. It is therefore of great interest to determine whether mutations in this gene can confer protection against NASH by reduced hepatocellular fatty acid influx.

Thus, the present inventors have discovered that operation of a four-protein plasma membrane complex is responsible for fatty acid and other monomeric lipid influx into mucosal cells from the intestinal lumen; and represents the key mechanism to lipid absorption. Surprisingly, it was found that inhibitors of phospholipase A (PLA), one of the four protein components of the complex, can be used as potent inhibitors of fat absorption, thus opening up new possibilities for therapeutical and non-therapeutical applications. The PLA inhibitors of the invention offer a new strategy for reducing fat absorption, and are thus novel tools for therapeutic treatment of obesity and associated diseases. In addition, said PLA inhibitors can be used for non-therapeutic body fat reduction and/or control for aesthetic reasons. Because the inventive PLA inhibitors preferably consist of naturally occurring compounds, they pose less health risks and are thus superior to other anti-obesity agents known in the prior art.

In a first aspect, the present invention relates to a phospholipase A inhibitor for use in a method of therapeutically reducing fat absorption in a subject. In one embodiment, said phospholipase A inhibitor is used for therapeutic reduction of body fat in a subject.

In one embodiment, the phospholipase A inhibitor of the invention is for treatment and/or prevention of obesity in a subject.

In one embodiment, the phospholipase A inhibitor can be a lysophospholipid-conjugate. The lysophosphoplipid can be chemically coupled to a bile acid. The bile acid can be ursodeoxycholate or deoxycholate. The lysophospholipid can be lysophosphatidylethanolamine or lysophosphatidylcholine.

In one embodiment, the phospholipase A inhibitor of the invention is orally administered. In one embodiment, the present invention envisages administering one or more anti-obesity agents prior to, simultaneously witg, or after the phospholipase A inhibitor. Said anti-obesity agents can be selected from the group of energy (re-)absorption inhibitors, peripheral modulators, CNS modulators, and herbal products.

In another embodiment, the subject treated with the phospholipase A inhibitor of the invention is also subjected to a diet and/or regular exercise.

In a second aspect, the present invention relates to a phospholipase A inhibitor for use in non-therapeutic fat reduction in a subject.

In a third aspect, the present invention relates to a method of non-therapeutic body fat reduction in a subject which comprises administering a phospholipase A inhibitor. In one embodiment, the method also comprises administering one or more anti-obesity agents prior to, simultaneously with, or after the phospholipase A inhibitor.

In a fourth aspect, the present invention relates to a pharmaceutical composition for use in treatment of obesity in a subject, comprising a lysophospholipid-conjugate and a pharmaceutically acceptable excipient. In one embodiment, the composition comprises one or more anti-obesity agents.

In a fifth aspect, the present invention relates to a lysophospholipid-conjugate for use in the manufacture of a medicament for the treatment of obesity in a subject.

In a six aspect, the present invention relates to the use of a lysophospholipid-conjugate for the treatment of obesity in a subject.

In a seventh aspect, the present invention relates to a kit for use in the treatment of obesity in a subject, comprising a lysophospholipid-conjugate and one or more anti-obesity agents.

In an eighth aspect, the present invention relates to a food product composition comprising a lysophospholipid-conjugate.

In a ninth aspect, the present invention relates to a method of diagnosing whether or not a subject, preferably a human, may be susceptible of NAFLD (non-alcoholic fatty liver disease), including NASH (non-alcoloholic steatohepatitis), comprising determining in a sample from said object whether a mutation is present in the PNPLA9 gene.

In a tenth aspect, the present invention relates to a method of diagnosing whether or not a subject, preferably a human, may be susceptible of NAFLD (non-alcoholic fatty liver disease), including NASH (non-alcoloholic steatohepatitis), comprising determining in a sample from said object whether a mutation is present in the CD36, FABP_{PM}, caveolin-1 and/or iPLA₂ß gene.

It must be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

The term "about" or "approximately" as used herein means within 20%, preferably within 10%, and more preferably within 5% of a given value or range. It includes, however, also the concrete number, e.g., about 20 includes 20.

The term "less than" or "greater than" includes the concrete number. For example, less than 20 means less than or equal to. Similarly, more than or greater than means more than or equal to, or greater than or equal to, respectively.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

In each instance herein any of the terms "comprising", "consisting essentially of" and "consisting of" may be replaced with either of the other two terms.

It should be understood that this invention is not limited to the particular methodology, protocols, material, reagents, and substances, etc., described herein and as such can vary. The terminology used herein is for the purpose of describing particular embodiments only, and is not intended to limit the scope of the present invention, which is defined solely by the claims.

All publications and patents cited throughout the text of this specification (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention. To the extent the material incorporated by reference contradicts or is inconsistent with this specification, the specification will supersede any such material.

### DESCRIPTION OF THE FIGURES

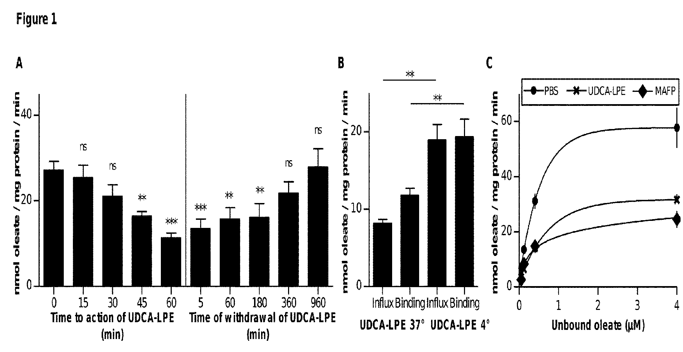

**Figure 1****. Inhibition of fatty acid influx by UDCA-LPE.** HepG2 cells pre-incubated with UDCA-LPE (100 µM, 1 h) were exposed to [³H]oleate:albumin (2:1 molar ratio). **A.** Time-dependent inhibition of influx and recovery after UDCA-LPE withdrawal. **B.** Inhibition of fatty acid influx and membrane binding by UDCA-LPE pretreatment at 37°C. C. Non-competitive inhibition of fatty acid influx by pretreatment with UDCA-LPE or the iPLA₂ß inhibitor MAFP (150 µM, 1 h). Treatment conditions were evaluated vs. time 0 or vs. indicated groups. Data represent mean ± SD. ** P < 0.01, *** P < 0.001 (n = 6).

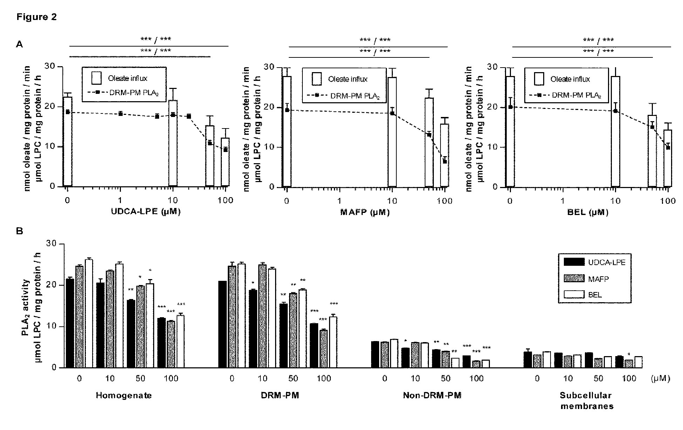

**Figure 2****. PLA₂ activity associated with the DRM-PM after pretreatment with UDCA-LPE, MAFP, and BEL. A.** Correlation of fatty acid influx inhibition and PLA₂ activity in DRM-PM fractions isolated from HepG2 cells after a 1-h treatment with increasing concentrations of UDCA-LPE or the iPLA₂ß inhibitors MAFP and BEL. **B.** PLA₂ activity in homogenates and subcellular fractions after treatment. Data represent mean ± SD. * P < 0.005, ** P < 0.01, *** P < 0.001 vs. time 0 (n = 6).

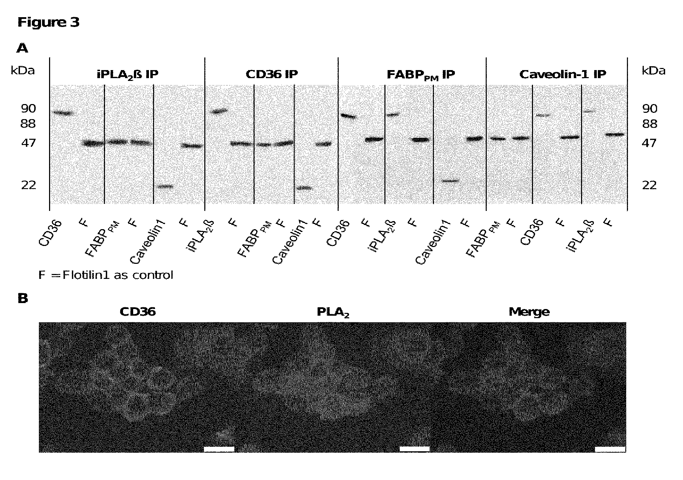

**Figure 3****. Identification of a fatty acid uptake complex in the DRM-PM consisting of CD36, FABPPM, caveolin1, and iPLA₂ß. A.** Immunoprecipitation (IP) of native DRM-PM from HepG2 cells using antibodies against each protein of the fatty acid uptake complex, followed by western blotting with antibodies against the other complex members. The DRM-PM (lipid raft) marker flotilin1 (F) served as a control. **B.** Immunofluorescence labeling of HepG2 cells with antibodies against iPLA₂ß and CD36 revealed their colocalization at the plasma membrane. Results are representative of four independent experiments. Scale bar = 20 µm.

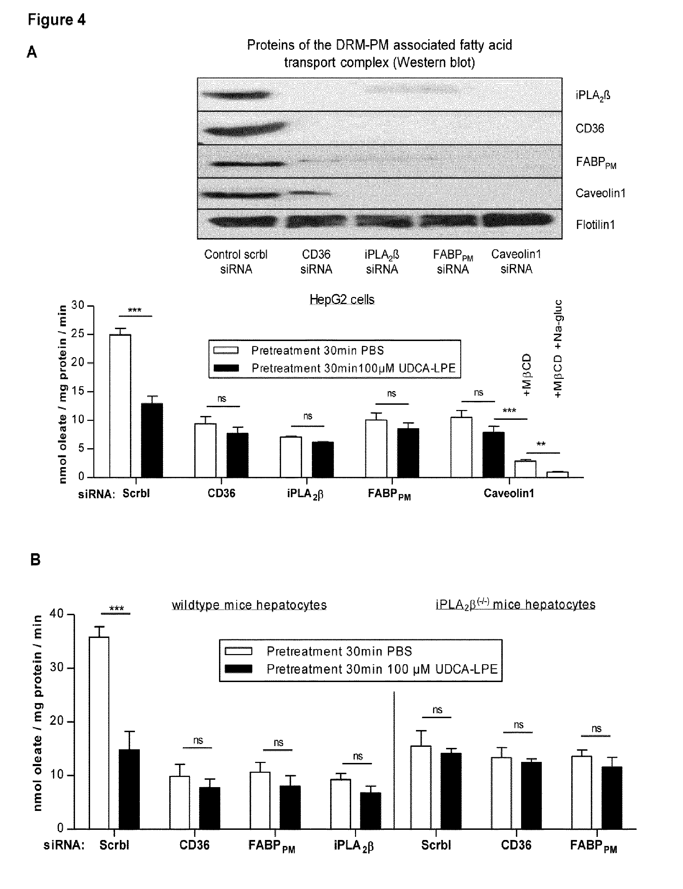

**Figure 4****. Functional analysis of fatty acid uptake complex components in HepG2 cells and mouse hepatocytes. A.** HepG2 cells treated for 16 h with siRNA against each of the four proteins of the fatty acid uptake complex (with scrambled siRNA used as control) revealed the simultaneous depletion of all components from the DRM-PM, and a concomitant inhibition of fatty acid influx and diminished sensitivity to UDCA-LPE pretreatment (100 µM, 1 h). Following pretreatment with CD36 siRNA, disruption of the DRM with MßCD (10 mM, 30 min) alone or in conjunction with Na-gluconate (Na-gluc; 133 mM), which prevented fatty acid protonation, resulted in further reductions in fatty acid influx. B. Effect of UDCA-LPE pretreatment on fatty acid influx in primary hepatocyte cultures derived from wild-type or iPLA₂ß-/ - mice. Cells were treated with the indicated siRNAs; UDCA-LPE pretreatment inhibited influx in wild-type but not iPLA₂ß-/ - cells transfected with scrambled siRNA. Knockdown of components of the uptake complex in wild-type hepatocytes resulted in a decrease in fatty acid influx and insensitivity to UDCA-LPE, while in iPLA₂ß-/ - hepatocytes a general decrease in influx was observed which was not further affected by siRNA knockdown or UDCA-LPE. Data represent mean ± SD. ** P < 0.01, *** P < 0.001 vs. control (n = 6). 18

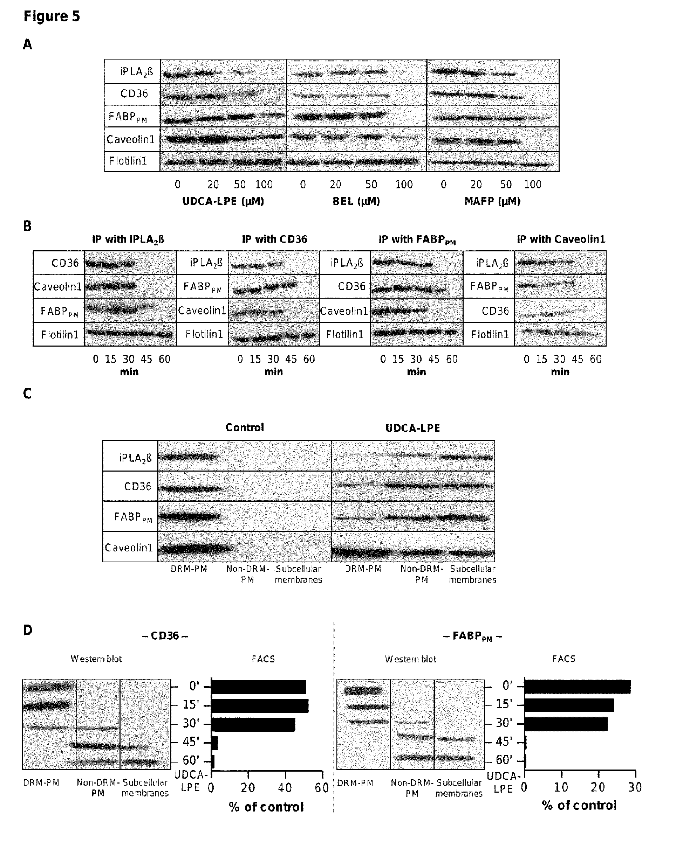

**Figure 5****. Displacement of fatty acid uptake complex components from the DRM-PM by UDCA-LPE pretreatment. A.** HepG2 cells were treated with indicated concentrations of UDCA-LPE, MAFP, and BEL for 30 min, followed by western blotting of DRM-PM isolated from these cells. **B.** Time-dependent complex disassembly after treatment of cells with UDCA-LPE (50 µM) as determined by subsequent immunoprecipitation (IP) of isolated DRM-PM with antibodies against each component, followed by western blotting with antibodies against other members of the complex as well as the DRM-PM (lipid raft) marker flotilin1 (F) as a control. **C.** Subcellular localization of complex components after treatment with UDCA-LPE (50 µM, 30 min) or PBS (control). D. Translocation of CD36 and FABPPM from DRM-PM to non-DRM-PM and subcellular membranes after treatment with UDCA-LPE (50 µM, 60 min) as determined by western blotting (left) and FACS analysis (right). Results are representative of four independent experiments.

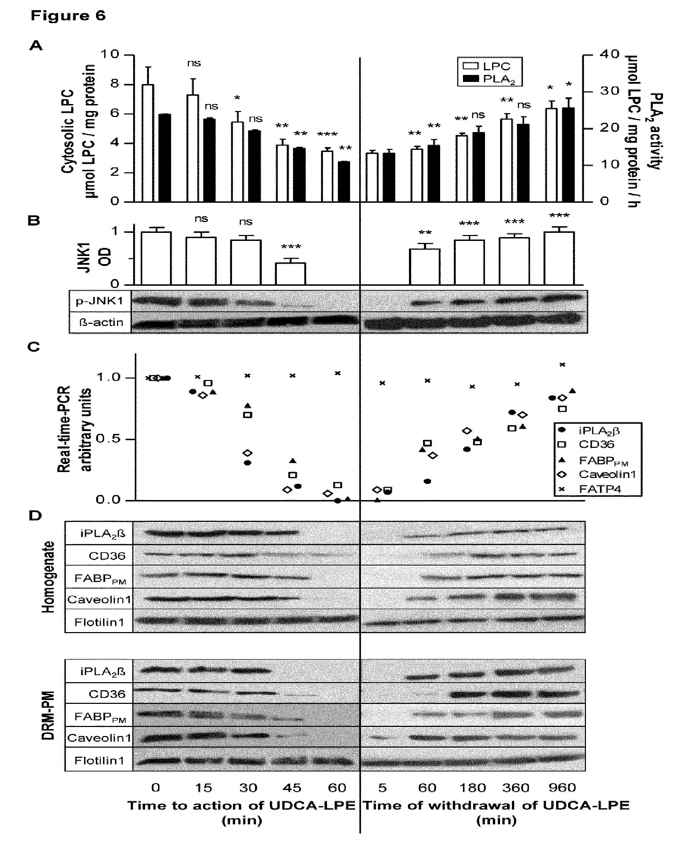

**Figure 6****. UDCA-LPE-mediated inhibition of synthesis of fatty acid uptake complex components and correlation with PLA₂ activity and cytosolic levels of LPC and p-JNK1.** Time-dependent inhibition and recovery of PLA₂ activity in HepG2 cell homogenates by treatment with UDCA-LPE (50 µM) in relation to cytosolic levels of A. LPC and B. p-JNK1, as determined by western blotting (with ß-actin as a loading control); and C. transcript expression of each member of the fatty acid uptake complex as determined by semi-quantitative RT-PCR. D. The presence of the components in the DRM-PM was assessed by western blotting, with FATP4 and flotilin1 serving as loading control and DRM-PM (lipid raft) marker, respectively. Data represent mean ± SD. * P < 0.05, ** P < 0.01, *** P < 0.001 vs. control (n = 6) for RT-PCR data; SD was < 5% in all cases (n = 6).

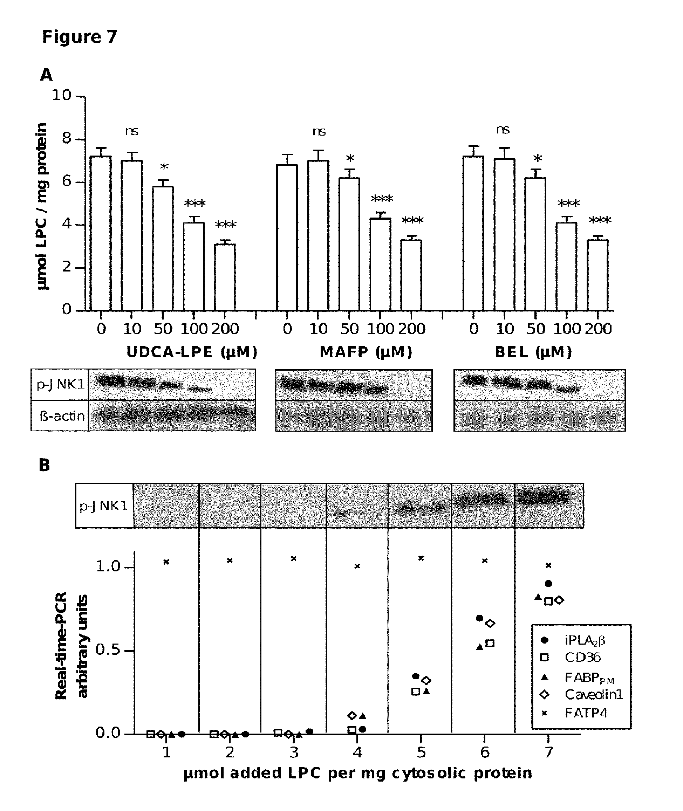

**Figure 7****. Cytosolic p-JNK1 concentration in relation to the synthesis of fatty acid uptake complex components. A.** Treatment of HepG2 cells with UDCA-LPE, MAFP, and BEL (30 min) revealed a concentration-dependent reduction in cytosolic levels of LPC (upper panels) and p-JNK1 (lower panels). ß- actin was used as a loading control for the western blot. B. Conditioned cytosolic extracts delipidated to eliminate interference from endogenous LPCs were incubated with indicated concentrations of LPC (1 h); a concentration-dependent increase in p-JNK1 levels was observed. *In vitro* transcription of the DRM-PM fatty acid uptake complex components in native HepG2 nuclear extracts exposed to the LPC-conditioned cytosolic samples was examined by semi-quantitative RT-PCR, with FATP4 serving as an internal negative control. A decrease in transcript levels of the complex components was observed at LPC concentrations = 5 µmol/mg cytosolic protein. Data represent mean ± SD. * P < 0.05, ** P < 0.01, *** P < 0.001 vs. control (n = 3); for semi-quantitative RT-PCR data SD was < 5% in all cases (n = 6). 19

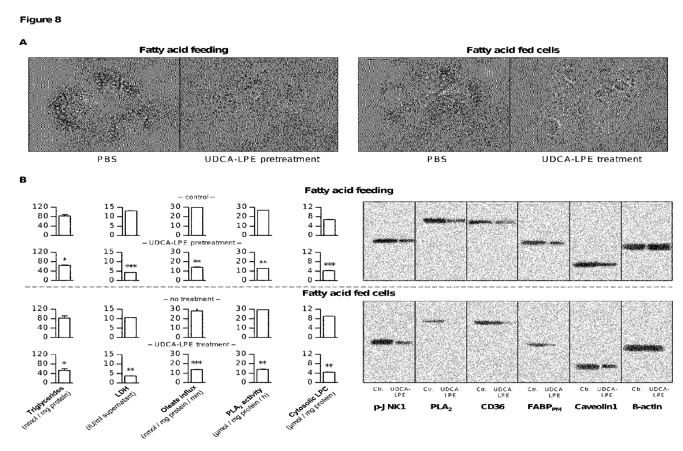

**Figure 8****. Abrogation of induced steatohepatitis by UDCA-LPE treatment.** In the fatty acid feeding condition, HepG2 cells were pretreated with UDCA-LPE (100 µM, 1 h) or PBS (control) and, then incubated for 3 h with a high concentration of free fatty acid. In the fatty acid fed condition, cells were first incubated with the high free fatty acids and then exposed to UDCA-LPE or PBS. **A.** Oil Red staining of HepG2 cells; and **B.** metabolic/inflammatory parameters and proteins (western blot). Under both conditions, triglyceride accumulation resulting from increased oleate uptake and LDH release, indicative of cell injury, were abolished, accompanied by a reduction in PLA₂ activity and cytosolic LPC concentration as well as decreased levels of p-JNK1 and members of the fatty acid uptake complex. ß-actin was used as a loading control for the western blot. Data represent mean ± SD. * P < 0.05, ** P < 0.01, *** P < 0.001 vs. control (n = 6).

### DETAILED DESCRIPTION

The present inventors have pioneered in finding that cellular fatty acid uptake is mediated by a four-protein plasma membrane complex, consisting of CD36, FABP_{PM}, caveolin-1 and iPLA₂ß. It is postulated that the four-protein fatty acid uptake complex may represent a ubiquitous entity in all cells, particularly those with a high fatty acid demand, because it ensures efficient as well as controlled fatty acid influx. The operation of a four-protein plasma membrane complex responsible for fatty acid and other monomeric lipid influx into mucosal cells from the intestinal lumen is thought to represent the key mechanism to lipid absorption. Based on this finding, the present inventors developed the concept of using PLA inhibitors for decreasing fat absorption. In particular, lysophospholipid-conjugates such das UDCA-LPE were found to act as PLA inhibitors which can inhibit operation of the four protein uptake complex, and, in addition, iPLA₂ß-mediated generation of lysophosphatidylcholine (LPC) which induces inflammation, apoptosis and cellular fatty acid uptake. Thus, the present inventors concluded that PLA inhibitors, in particular lysophospholipid-conjugates such as UDCA-LPE, can act to inhibit fat absorption, and besides to suppress inflammation and apoptosis. This inhibition takes place in the gastrointestinal tract, where fatty acids, monoglycerides, lysophospholipids, and cholesterol, all utilize the four protein fatty acid uptake complex. Thus, PLA inhibitors of the invention can be used to inhibit fat absorption into intestinal mucosal cells. Furthermore, it is speculated that fatty acid receptor structures on tongue epithelial cells and enterochromaffine cells of the duodenum may involve the same proteins, at least CD36 and iPLA₂ß. They control by neuroendocrine stimulation taste, appetite and pancreatic secretion which is inhibited by exposure to PLA inhibitors.

Thus, PLA inhibitors of the invention, in particular lysophospholipid-conjugates such as UDCA-LPE, can be used to control fat absorption, digestion and appetite. As the PLA inhibitors of the invention are preferably lysophospholipid-conjugates, i.e. are composed of naturally occurring components, they are well-tolerated and do not elicit severe side-effects as many other anti-obesity agents do. In addition, the PLA inhibitors of the invention can be conveniently administered orally, and even added to foods. Thus, they are promising new tools for treating obesity and associated diseases. Furthermore, due to their innocuousness, they are also well-suited for non-therapeutic body fat-reduction and/or control for aesthetic reasons. As a major advantage, instead of eliciting severe side-effects, the PLA inhibitors of the invention preferably even reduce inflammation and apoptosis.

The PLA inhibitors are envisaged for use in a method of reducing fat absorption in a subject. Without wishing to be bound by theory, it is thought that they do so by causing disassembly of the fatty acid uptake complex, which iPLA₂ is a part of. In consequence, the PLA inhibitors of the invention are also envisaged for therapeutic reduction of body fat in a subject. It is thought that by decreasing overall fat absorption, the PLA inhibitors of the invention promote reduction of fat depots. Notably, reduction of body fat in the obese is typically associated with a reduction of body weight. Thus, the PLA inhibitor of the invention is also envisaged for therapeutic reduction of body weight in obesity treatment. However, body weight reduction may also depend on other factors, e.g. water and muscle content.

The term "obesity" in general refers to an abnormal or excessive fat accumulation that presents a risk to health. A crude population measure of obesity in adults is the body mass index (BMI), a person's weight (in kilograms) divided by the square of his or her height (in meters). The term "obesity" is herein adopted to describe a condition characterized by a BMI ≥ 30 kg/m² or a BMI ≥ 27 kg/m² combined with at least one comorbidity as described herein in adults. For adolescents, "obesity" refers to a condition characterized by two standard deviations body mass index for age and sex from the World Health Organization (WHO) growth reference for school-aged children and adolescents. The term "obesity" thus implies a medical indication for treatment. Thus, in one aspect, the present invention relates to a PLA inhibitor for use in therapeutic reduction of body fat in a subject. In particular, the PLA inhibitor is envisaged for use in treatment and/or prevention of obesity in a subject.

However, the PLA inhibitor of the invention can as well be used for non-therapeutic body fat reduction in non-obese subjects, i.e. "normal-weight" subjects having a BMI ≤ 25 kg/m² or "overweight" subjects having a BMI ≥ 27 kg/m² but ≤ 30 kg/m² and without any obesity-associated health implications (i.e. co-morbidities). This use is exclusively for aesthetic or cosmetic reasons and not based on a medical indication. Also, body fat reduction (and/or control) in non-obese subjects might involve body weight reduction (and/or control). However, e.g. in case of simultaneous physical training, non-therapeutic body fat reduction and/or control may not be accompanied by overall weight reduction due to muscle growth etc. It is also envisaged to use the PLA inhibitor for non-therapeutic body fat control. "Control" in this context means maintenance at a desired level, e.g. when no further body fat reduction is intended but additional fat accumulation is desired to be prevented. Thus, in a further aspect, the present invention relates to a PLA inhibitor, in particular a lysophospholipid-conjugate such as UDCA-LPE, for use in non-therapeutic body fat reduction and/or control in a subject.

It is to be understood that the PLA inhibitor of the invention, which is in particular a lysophospholipid-conjugate such as UDCA-LPE, is also envisaged for therapeutic treatment of obesity-associated diseases. The term "co-morbidity" and "obesity-associated disease" are used interchangeably herein and include obstructive sleep apnea, greater predisposition to respiratory infections, increased incidence of bronchial asthma, and Pickwickian syndrome (obesity hypoventilation syndrome), endometrial, prostate, colon, breast, gall bladder, and possibly lung cancer, depression, coronary artery disease, essential hypertension, left ventricular hypertrophy, cor pulmonale, obesity-associated cardiomyopathy, accelerated atherosclerosis, pulmonary hypertension of obesity, stroke, idiopathic intracranial hypertension, meralgia paresthetica, pregnancy-related hypertension, fetal macrosomia, pelvic dystocia, wound infection, postoperative pneumonia, deep venous thrombosis, and pulmonary embolism, stress incontinence, gall bladder disease (cholecystitis, cholelithiasis), nonalcoholic steatohepatitis (NASH), fatty liver infiltration, and reflux esophagitis, osteoarthritis, coxa vera, slipped capital femoral epiphyses, Blount disease and Legg-Calvé-Perthes disease, chronic lumbago, type 2 diabetes mellitus, prediabetes, metabolic syndrome, dyslipidemia, anovulation, early puberty, infertility, hyperandrogenism, polycystic ovaries, hypogonadotropic hypogonadism, intertrigo (bacterial and/or fungal), acanthosis nigricans, hirsutism, increased risk for cellulitis and carbuncles, venous varicosities, lower extremity venous and/or lymphatic edema, and reduced mobility and difficulty maintaining personal hygiene.

Phospholipases (PL) are abundant throughout the prokaryotic and eukaryotic kingdom and constitute a group of lipolytic enzymes that share the ability to hydrolyze one or more ester linkages in phospholipids. PL are typically classified based on their site of action; phospholipases A (PLA) cleave in the hydrophobic diacylglycerol moiety. PLAs can be further defined by their positional specificity, i.e. preference for the acyl group attached to position 1 or 2 of the glycerol backbone, as PLA₁ and PLA₂, respectively; PLBs have both PLA₁ and PLA₂ activity, i.e., little or no positional specificity.

Preferably, the PLA inhibitor of the invention is a PLA₂ inhibitor. It is particularly preferred that the inhibitor of the invention inhibits iPLA₂ (Ca2+ independent PLA₂, also referred to as group VI PLA₂), comparable to the compounds evaluated in the appended examples. However, it is also envisaged that the inventive PLA inhibitor inhibits secreted PLA₂, in particular secreted pancreatic PLA₂.

The term "inhibiting" in all its grammatical forms as used herein means "abolishing or decreasing a physiological function". Preferably, an inhibitor of PLA decreases or abolishes ability of a PLA to hydrolyze fatty acids. In addition, it is envisaged that the inhibitor of the invention abolishes or reduces fat absorption, presumably by impairing PLA function as a part of the four protein fatty acid uptake complex. Without wishing to be bound by a specific theory, it is thought that the PLA inhibitor of the invention promotes disassembly of the four protein complex, thereby inhibiting fat influx.

The terms "lipid" and "fat" are used interchangeably throughout the specification.

The term "four protein complex" and "four protein fatty acid uptake complex" and "fatty acid uptake complex" are used interchangeably throughout the specification and refer to a protein complex comprising CD36, FABPPM, caveolin-1 and iPLA₂ß which presumably mediates cellular uptake of fatty acids monoglycerides, lysophospholipids, and cholesterol. "CD36" (Cluster of Differentiation 36), also known as FAT (fatty acid translocase), FAT/CD36, (FAT)/CD36, SCARB3, GP88, glycoprotein IV (gpIV), and glycoprotein IIIb (gplllb), is an integral membrane protein found on the surface of many cell types in vertebrate animals. "FABPPM" or plasma membrane fatty-acid-binding protein is a carrier proteins for fatty acids and other lipophilic substances. "Caveolin-1" is a scaffolding protein and the main component of the caveolae plasma membranes found in most cell types. "iPLA₂ß" (PLA2G6) is the Ca²⁺-independent phospholipase A enzyme which catalyzes hydrolyzation of phospholipids into fatty acids and other lipophilic substances.

The terms "uptake" and "absorption" and "influx" are used interchangeably herein.

In principle, in order to identify potential PLA inhibitors, the person skilled in the art can use the three dimensional structure of PLA or its active site in order to predict which compounds might be inhibitors. Various methods for determining possible ligands have been reviewed in Anderson AC et al. Chem Biol. 2003; 10: 787-797. In general, once the target structure has been determined, e.g., by X-ray crystallography, NMR, or homology modeling, computer algorithms can be used to position compounds or fragments thereof from a database into a selected region of the structure. These compounds can be scored and ranked based on their steric and electrostatic interactions with the target site. Such compounds are useful for example as a lead for the development of further analogues, which in turn may have enhanced inhibitory potential or other beneficial therapeutic properties. On the other hand, the selected compound may bind to a site of the target other than known ligands. Lead compounds can be improved using the 3-D structure of the complex of the lead compound and its biological target. The activity of the selected compound can further be tested with the biochemical assays described herein.

E.g., the structure of human iPLA₂ has been determined by Hsu HY et al. J Biol Chem 2009; 284:23652-23661 using hydrogen/deuterium exchange mass spectroscopy and homology modeling. It is envisaged that the predicted structure of human iPLA₂ allows an evaluation of potential iPLA₂ inhibitors. Other authors have determined the 3-D structure of secreted pancreatic phospholipases A₂ (see Dennis EA. J. Biol. Chem. 1994; 269:13057.) Accordingly, computer-aided methods can be used to identify candidate inhibitors for PLA Said methods are further classified into at least three categories: inspection, virtual screening, and *de novo* generation. In the first category, inspection, known molecules that bind the site, such as substrates or cofactors, are modified to become inhibitors based on maximizing complementary interactions in the target site. Initially, the crystal structure is solved in the presence of a substrate, cofactor, or drug lead. Then, modifications to direct the small molecule toward being a potent inhibitor are designed *in silico* based on the interactions of the molecule with the target site. The newly designed compounds are then scored for binding using evaluative scoring algorithms available in virtual screening methods.

In virtual screening, databases of available small molecules are docked into the region of interest *in silico* and scored based on predicted interactions with the site, e.g. shape complementarity or estimated interaction energy. For *de novo* generation small fragments of molecules, such as benzene rings, carbonyl groups, amino groups, etc., are positioned in the site, scored, and linked *in silico.* Some programs, e.g., LUDI, are capable of docking fragments of compounds as well as entire compounds, and can thus be used for virtual screening and *de novo* generation, respectively. Suitable screening tools that can be used to find PLA₂ inhibitors include DOCK, FlexX, FlexE, LUDI and Legend (see Anderson AC et al. (loc cit.) for other suitable programs).

In view of the fact that PLA was crystallized, the known PLA crystals can also be used in X-ray crystallography-driven screening technique that combines the steps of lead identification, structural assessment, and optimization such as described for example in Nienaber VL et al. Nat Biotechnol. 2000; 18(10): 1105-1058. This crystallographic screening method (named CrystaLEAD) has been used to sample large compound libraries and detecting ligands by monitoring changes in the electron density map of the crystal relative to the unbound form. The electron density map yields a high-resolution picture of the ligand-enzyme complex that provides key information to a structure- directed drug discovery process. The bound ligand is directly visualized in the electron density map. Ligands that bind way off the target site may be eliminated. The above described methods can be combined with state-of-the-art laboratory data collection facilities including CCD detectors and data acquisition robotics.

The above-mentioned methods can be used to assess the inhibitory potential of a given compound on PLA, and/or to identify candidate PLA inhibitors from a library. Once a compound has been identified as a candidate inhibitor by the above methods, its inhibitory effect on PLA may be tested by the method described in the appended example.

As stated herein, the PLA inhibitor of the present invention, which is in particular a lysophospholipid-conjugate such as UDCA-LPE, is preferably a PLA₂ inhibitor. "PLA₂" in this context generally includes all types of PLA₂, including without limitation secreted and cytosolic forms. The inhibitory effect of a specific compound on PLA₂ can be easily determined by the skilled person by using routine methods as described in the appended examples. In addition, various further assays, including enzyme assays and cell-based assays, for testing PLA₂ activity have been described, e.g., by Ono T et al. Biochem J. 2002; 363: 727-735. Exemplary enzyme assays for assessing, e.g., human cytosolic PLA₂ activity, include, without limitation, the phosphatidylcholine (PC)/Triton assay, the chromogenic assay and the PC/DOG assay. Human sPLA₂ activity can for example be measured using diheptanoyl thio-PC as a substrate according to Reynolds LJ et al. Anal Biochem. 1992; 204: 190-197. In principle, all methods provide phospholipid-substrates and assess their conversion due to PLA activity. Cell-based assays for assessing human PLA₂ activity employ, for example, thrombin-stimulated platelets, calcium-ionophore-stimulated monocytes (e.g., THP-1, see Ono et al., loc cit.) or interleukin-1α stimulated human mesangial cells. Fatty acids including arachnidonic acid, prostaglandin or leukotriene from stimulated cells are extracted, and quantified, e.g. by HPLC or by using radioimmunoassay kits or enzyme immunoassay kits. Ready-to-use kits for determining enzyme activity of PLA in the presence of a candidate inhibitor are also commercially available, e.g., from Cayman Chemicals (sPLA₂ (Type V) Inhibitor Screening Assay Kit) and Invitrogen (EnzChek® Phospholipase A₂ Assay Kit).

The present inventors have discovered that in particular lysophospholipid-conjugates are potent PLA inhibitors. Thus, in one embodiment, the PLA inhibitor of the invention is a lysophospholipid-conjugate.

The novel inhibitors according to the present invention are envisaged for treatment of a subject, preferably a mammal, which can be, for instance, a mouse, rat, guinea pig, hamster, rabbit, dog, cat, or primate. Preferably, the subject is a human.

The present inventors were the first to provide lysophospholipid-conjugates for inhibition of fat absorption as described herein. The term "lysophospholipid-conjugate" as used herein refers to lysophospholipids that are chemically coupled to a carrier. The term "chemically coupled" means, e.g., covalently coupled. However, any other chemical bond is also conceivable. Lysophospholipids (LPL) are naturally derived from phospholipids, such as phosphatidylcholine (PC), phosphatidylethanolamine (PE), phosphatidic acid (PA), phosphatidylserine (PS), phosphatidylinositol (PI) or other phospholipids. Lysophospholipids can, for example, be the result of phospholipase A-type enzymatic activity on phospholipids. Phospholipids are typically composed of two fatty acids, a glycerol unit, a phosphate group and a polar molecule such as, e.g., choline in phosphatidylcholine, or ethanolamine in phosphatidylethanolamine. In contrast, lysophospholipids typically comprise only one fatty acid. In general, any lysophospholipid can be used in the lysophospholipid-conjugate according to the present invention. Suitable lysophospholipids include, but are not limited to, lysophosphatidate, lysophosphatidylethanolamine, lysophosphatidylcholine, lysophosphatidylserine, lysophosphatidylinositol, lysophosphatidylinositolphosphate, lysophosphatidylinositolbisphosphate, lysophosphatidylinositoltriphosphate. In one preferred embodiment, the lysophospholipid is lysophosphatidylethanolamine or lysophosphatidylcholine. It is also envisaged that a phospholipid-conjugate can be used for the treatment of the diseases described herein. The phospholipid could, for example, be phosphatidic acid (phosphatidate), phosphatidylethanolamine (PE), phosphatidylcholine (PC), phosphatidylserine (PS), phosphatidylinositol (PI), phosphatidylinositol phosphate (PIP), phosphatidylinositol bisphosphate (PIP2) or phosphatidylinositol triphosphate (PIP3).

Bile acids are preferred carriers. However, it is also envisaged that other carriers may be used. The selected carrier can be, or can be derived from, a naturally occurring component. Bile acids are steroid acids that are naturally found in the mammalian bile. Primary bile acids, such as, e.g. cholate, are naturally synthesized in the liver and secreted into the lumen of the intestine, where intestinal bacteria chemically convert them to form the secondary bile acids such as, e.g., deoxycholic acid. Suitable bile acids include, but are not limited to, cholic acid, chenodeoxycholic acid, deoxycholic acid, lithocholic acid, glycocholic acid, taurocholic acid, ursodeoxycholic acid. In one preferred embodiment, the bile acid is ursodeoxycholic acid (UDCA) or deoxycholic acid (DCA). It is also envisaged that chemically modified bile acids can be used as carriers in the present invention.

In general, any lysophospholipid and any carrier can be combined to yield the lysophospholipid-conjugate of the invention. The lysophospholipid-conjugate should preferably have capabilities that are similar to the capabilities of the lysophospholipid-conjugate which are evaluated in the appended examples. For example, the lysophospholipid-conjugate of the present invention is preferably capable of inhibiting PLA activity.

In view of the above, preferred embodiments of the invention thus involve the use of UDCA-LPE, UDCA-LPC, DCA-LPE and DCA-LPC for the treatment of the diseases described herein. By inhibiting PLA, it is contemplated that PLA inhibitors, which are preferably lysophospholipid-conjugates, of the invention inhibit fat absorption. Accordingly, it is envisaged that the PLA inhibitor, preferably a lysophospholipid-conjugate such as UDCA-LPE, of the invention is used for therapeutic body fat reduction, and in particular for treating obesity, preferably in a mammal. However, the PLA inhibitor is also envisaged for non-therapeutic body fat reduction and/or control, as stated elsewhere herein.

### Mode of action

Without wishing to be bound by specific theory, it is thought the inhibitor of the invention acts in several ways. As set our herein, the present inventors have discovered that cellular fatty acid uptake is mediated by a four-protein plasma membrane complex, consisting of CD36, FABPPM, caveolin-1 and iPLA₂ß. Most of the fat in the human diet is present in the form of triacylglycerol (TAG), which consists of three fatty acids linked to glycerol. In the digestive tract, TAG is hydrolyzed by the enzyme lipase, to release free fatty acids and monoglycerides, which are thought to be absorbed by action of the four-protein fatty acid uptake complex. The PLA inhibitor, in particular a lysophospholipid-conjugate such as UDCA-LPE, is thought inhibit iPLA₂ß which leads to disintegration of the uptake complex, thereby suppressing absorption of fatty acids, monoglycerides, lysophospholipids, and cholesterol. Furthermore, generation of lysophosphatidylcholine (LPC) is inhibited, which is associated with JNK1 phosphorylation which in turn stimulates inflammation, apoptosis and cellular fatty acid uptake. Thus, the PLA inhibitor of the invention, in particular a lysophospholipid-conjugate such as UDCA-LPE, is thought to inhibit fatty acid uptake and to suppress inflammation and apoptosis. Moreover, it is also thought that said PLA inhibitor can block secreted pancreatic phospholipases, thereby impairing fat digestion. As an additional advantage, undigested phospholipids may in distal parts of the intestine exert mucosal protective properties.

Furthermore, fatty acid receptor structures on tongue epithelial cells and enterochromaffine cells of the duodenum may involve the same proteins, at least CD36 and iPLA₂ß. Influx of the PLA inhibitor of the invention may allow the interaction with the inner plasma membrane localized iPLA₂ß. This is thought to displace CD36 and, thus, the events controlling taste, appetite and pancreatic secretion through CD36 and neuroendocrine stimulation are interrupted. Thus, exposure of tongue epithelial cells by topical application of a PLA inhibitor, in particular a lysophospholipid-conjugate such as UDCA-LPE, may help to decrease appetite. This may also apply to any other inhibitor of the four-protein uptake complex as long as it is operative on tongue epithelium.

Further down in the gastrointestinal tract, enterochromaffine cells of the duodenal wall respond to fatty acid exposure by releasing pancreozymine/cholecystokinin (PZ/CCK) to promote enzymatic food digestion. Inhibition of these fatty acid responsive mechanisms by PLA inhibitor of the invention is thought to inhibit digestion. The PLA inhibitor of the invention may also affect release of hormones involved in endocrine pancreatic secretion which may be used to reverse insulin resistance.

With regard to obesity-associated non-alcoholic steatohepatitis (NASH), the present inventors have found evidence that a fraction of UDCA-LPE may be translocated across the mucosal cells and targeted in the organism to hepatocytes. Here it could exert lipolytic, antiapoptotic and antiinflammatory effects to fight NASH.

In conclusion, the PLA inhibitor of the invention, in particular a lysophospholipid-conjugate such as UDCA-LPE, is thought to interfere with fat absorption at different points of action along the gastrointestinal tract starting at CD36 receptors in tongue epithelial cells and ending at the four protein fatty acid uptake complex in enterocytes in the intestine.

### Pharmaceutically acceptable salts

For the purpose of the invention the active compound as defined above also includes the pharmaceutically acceptable salt(s) thereof. The phrase "pharmaceutically or cosmetically acceptable salt(s)", as used herein, means those salts of compounds of the invention that are safe and effective for the desired administration form. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc. If the PLA inhibitor of the invention is a lysophospholipid-conjugate, and in particular UDCA-LPE, it is in particular envisaged to be used in its Na⁺ salt form.

The use of salt formation as a means of varying the properties of pharmaceutical compounds is well known and well documented. Salt formation can be used to increase or decrease solubility, to improve stability or toxicity and to reduce hygroscopicity of a drug product. There are a wide range of chemically diverse acids and bases, with a range of pKa values, molecular weights, solubilities and other properties, used for this purpose. Of course, any counter ions used in pharmaceuticals must be considered safe, and several lists of pharmaceutically approved counter ions exist, which vary depending on the source. Approved salt formers can e.g. be found in the Handbook of Pharmaceutical Salts (Stahl PH, Wermuth CG, editors. 2002. Handbook of pharmaceutical salts: Properties, selection and use. Weinheim/Zurich: Wiley-VCH/VHCA.). Thus, the present invention also comprises the use of pharmaceutically acceptable salts of the PLA inhibitor of the invention for the uses described herein.

### Chemical modification

The PLA inhibitors of the invention may also be chemically modified. Generally, all kind of modifications are contemplated as long as they preferably do not abolish the advantageous effects of the PLA inhibitor of the invention, i.e. the chemically modified PLA inhibitors should preferably have capabilities which are comparable to the capabilities of the compounds which were evaluated in the appended examples. That is, the chemically modified PLA inhibitor of the invention should be capable of reducing fat absorption in a subject and thus should be able to elicit a cosmetic and/or therapeutic effect in a subject.

It may be necessary, for reasons of resistance to degradation, to employ a protected form of the PLA inhibitor. The nature of the protecting group must obviously be a biologically compatible form. Thus, the invention also features the PLA inhibitor of the invention in a protected or unprotected form.

### Therapeutic and cosmetic effect

In the context with the present invention the term "therapeutic effect" in general refers to the desirable or beneficial impact of a treatment, e.g. amelioration or remission of the disease manifestations. The term "manifestation" of a disease is used herein to describe its perceptible expression, and includes both clinical manifestations, hereinafter defined as indications of the disease that may be detected during a physical examination and/or that are perceptible by the patient (i.e., symptoms), and pathological manifestations, meaning expressions of the disease on the cellular and molecular level.

The therapeutic effect of the uses and methods described herein is additionally detectable by all methods and approaches that are established for indicating a therapeutic effect in the treatment of obesity and associated conditions (such as NASH). Methods for monitoring the therapeutic effect of the PLA inhibitor, in particular a lysophospholipid such as UDCA-LPE, include, but are not limited to assessing body weight and determining the BMI, and determining body fat percentage by, e.g., hydrostatic weighing, whole-body air displacement plethysmography, near-infrared interactance, dual energy X-ray absorptiometry (DXA), body average density measurement, Bioelectrical impedance analysis (BIA), Anthropometric methods (skinfold/pinch test, ultrasound, estimation from BMI). Additionally or alternatively it is also possible to evaluate the general appearance of the respective patient (e.g., fitness, well-being) which will also aid the skilled practitioner to evaluate whether a therapeutic effect has been elicited. The skilled person is aware of numerous other ways which are suitable to observe a therapeutic and/or cosmetic effect of the compounds of the present invention.

The term "therapeutic treatment" in all its grammatical forms includes therapeutic or prophylactic treatment. A "therapeutic or prophylactic treatment" comprises prophylactic treatments aimed at the complete prevention of clinical and/or pathological manifestations or therapeutic treatment aimed at amelioration or remission of clinical and/or pathological manifestations. The term "therapeutic treatment" thus also includes the amelioration or prevention of obesity and associated conditions, such as NASH.

The term "cosmetic effect" is used herein to describe the desired advantageous impact of the PLA inhibitor of the invention with regard to appearance, which is associated with loss or control of body fat, and preferably an enhancement of body shape and definition. It is to be understood that the non-therapeutic cosmetic treatment of a subject with the PLA inhibitors of the invention, in particular lysophospholipid-conjugates such as UDCA-LPE, is for aesthetic reasons only and is exclusively accomplished in subjects that do not exhibit an amount of body fat that significantly increases health risks. Non-therapeutic, cosmetic treatment with the PLA inhibitor of the invention may also induce loss of weight and/or serve for weight control in order to prevent a (non-pathological) body fat accumulation and/or gain of weight. The cosmetic effect can in general be assessed with the methods described in the context of the therapeutic effect of the PLA inhibitor of the invention.

### Dose

The exact dose of PLA inhibitor of the invention will depend on the purpose of the treatment (e.g. remission maintenance vs. acute flare of disease, therapeutic vs. cosmetic (i.e. non-therapeutic) treatment), and will be ascertainable by one skilled in the art using known techniques. As is known in the art and described above, adjustments for route of administration, age, body weight, general health, sex, diet, time of administration, drug interaction and the severity of the condition may be necessary, and will be ascertainable with routine experimentation by those skilled in the art.

### Anti-obesity agents

It is also envisaged that the PLA inhibitor of the invention, which is in particular a lysophospholipid-conjugate such as UDCA-LPE, is used in combination with other anti-obesity agents.

Anti-obesity agents for use in accordance with the present invention have been reviewed, i.a., in Witkamp RF. Pharm Res. Aug 2011; 28(8): 1792-1818 and include, without limitation, energy (re-)absorption inhibitors, peripheral modulators, CNS modulators, and herbal products.

Energy (re-)absorption inhibitors are preferably locally acting compounds which reduce the uptake of energy-rich molecules from the GI tract. Exemplary energy (re-)absorption inhibitors include, without limitation, pancreatic lipase inhibitors (e.g., orlistat, cetilistat), chitosan, glucomannan, psyllium seeds, inhibitors of Microsomal Triglyceride transfer Protein (MTP), inhibitors of Diacylglycerol O-acyltransferase (DGAT), inhibitors of 2-acylglycerol O-acyltransferase 2 (MGOT), and inhibitors of low-affinity sodium-dependent glucose co-transporters (SGLT2).

Peripheral modulators interfere with the perception of nutrient sensing within the GI tract and/or with signals transmitted to the brain. Exemplary peripheral modulators include, without limitation, GPR119 agonists, CB1 neutral and/or partial agonists (e.g. 7-carbamoylmethyl-pyrazole-carboximide derivatives), amylin analogues (e.g. pramlintide), PYY analogues, PP analogues/Y4 agonists (e.g. obinepitide), GLP-1 analogues (e.g. exenatide, liraglutide), oxyntomodulin analogues, leptin analogues (e.g. alone or in combination, e.g. metreleptin-pramlintide combination), cholecystokinin-1 (CCK) agonists, and Ghrelin blockers.

CNS modulators interfere with signal processing within the CNS, for example by interfering with neurotransmitter concentrations or activity. Exemplary CNS modulators include, without limitation, dopamine/norepinephrine/serotonin reuptake inhibitors (e.g., sibutramine; bupropion; tesofensine; mazindol), GABA stimulators (e.g. topiramate (also in combinations), zonisamide (also in combinations) dopamine/norepinephrine/serotonin release stimulators (e.g. phentermine, also in combination with topiramat; phenmetrazine; phendimetrazine, amfepramone; methamphetamine; benzphetamine), serotonin-receptor (5-HT_{2c}) agonists (e.g. lorcaresin), serotonin-receptor (5-HT₆) agonists, MC4-receptor agonists, MCH-receptor type 1 antagonists, Histamine (H3-) receptor antagonists, NPY (Y5) receptor antagonists (e.g., velneperit), AgRP inhibitors, PTP1 B inhibitors (e.g. trodusquemine),

Herbal products for use as anti-obesity agents in accordance with the present invention include saponins, polyphenols, terpenes, CT-II, AR25 (Exolise), green tea extract, epigallocatechin gallate (EGCG), *Salacia reticulata* extract (SRHW), grape seed extract (GSE), soy protein and its isoflavones, *Morinda citrifolia* (noni) fruit extract, *Momordica charantia* (bitter gourd) extract, *Centella asiatica* (Asian pennywort) extract, Perilla oil, *Hibiscus sabdariffa* extract, lemon polyphenols, *Ganoderma lucidum* extract, *Hoodia pilifera* extract, *Hoodia gordonii* extract, *Caralluma fimbriata* extract, *Catha edulis* (khat) extract, *Garcinia cambogia* extract, yerba mate exract, *Coleus forskohlii* extract, *Betula alba* extract. These and other herbal products suitable for obesity-treatment have been reviewed in Gooda Sahib N et al. Sci World J. 2012;2012:436039. The person skilled in the art will readily acknowledge that the aforementioned herbal products could as well be classified according to their function as the anti-obesity agents mentioned herein.

The anti-obesity agents mentioned herein are contemplated for administration before, simultaneously to, or after the PLA inhibitor of the invention, which is in particular a lysophospholipid-conjugate such as UDCA-LPE.

The may also be applicable for use in the pharmaceutical composition, the kit and/or the method of the present invention.

### Composition

The PLA inhibitor of the invention, in particular a lysophospholipid-conjugate such as UDCA-LPE, can also be used as part of a pharmaceutical composition. Thus, a further embodiment of the invention is the use of the PLA inhibitor of the invention, in particular UDCA-LPE, for the manufacture of a pharmaceutical composition for treatment and/or prevention of obesity and optionally also of obesity-associated conditions. It is to be acknowledged that the embodiments described in the context of the use of a PLA inhibitor according to the present invention are equally applicable to the uses of the pharmaceutical composition comprising said PLA inhibitor, *mutatis mutandis.* The pharmaceutical composition may further comprise a pharmaceutically acceptable excipient. Processes known *per se* for producing medicaments are indicated in Forth, Henschler, Rummel (1996) Allgemeine und spezielle Pharmakologie und Toxikologie, Urban & Fischer.

Pharmaceutical compositions of the invention comprise a therapeutically effective amount of the PLA inhibitor of the invention, in particular a lysophospholipid-conjugate such as UDCA-LPE, By "therapeutically effective amount" is meant an amount of the PLA inhibitor of the invention, in particular UDCA-LPE, that elicits a therapeutic effect as described herein.

### Pharmaceutically acceptable excipients

The pharmaceutical composition may be administered with a pharmaceutically acceptable excipient to a patient, as described herein. In a specific embodiment, the term "pharmaceutically acceptable" means approved by a regulatory agency or other generally recognized pharmacopoeia for use in animals, and more particularly in humans. Accordingly, the pharmaceutical composition may further comprise pharmaceutically acceptable excipients.

Pharmaceutically acceptable excipients include, but are not limited to ddiluents (fillers, bulking agents, e.g. lactose, microcrystalline cellulose), disintegrants (e.g. sodium starch glycolate, croscarmellose sodium), binders (e.g. PVP, HPMC), lubricants (e.g. magnesium stearate), glidants (e.g. colloidal SiO₂), solvents/co-solvents (e.g. aqueous vehicle, Propylene glycol, glycerol), buffering agents (e.g. citrate, gluconates, lactates), preservatives (e.g. Na benzoate, parabens (Me, Pr and Bu), BKC), anti-oxidants (e.g. BHT, BHA, Ascorbic acid), wetting agents (e.g. polysorbates, sorbitan esters), anti-foaming agents (e.g. Simethicone), thickening agents (e.g. methylcellulose or hydroxyethylcellulose), sweetening agents (e.g. sorbitol, saccharin, aspartame, acesulfame), flavouring agents (e.g. peppermint, lemon oils, butterscotch, etc), humectants (e.g. propylene, glycol, glycerol, sorbitol). The person skilled in the art will readily be able to choose suitable pharmaceutically acceptable excipients, depending, e.g., on the formulation and administration route of the pharmaceutical composition.

A non-exhaustive list of exemplary pharmaceutically acceptable excipients includes (biodegradable) liposomes; microspheres made of the biodegradable polymer poly(D,L)-lactic-coglycolic acid (PLGA), albumin microspheres; synthetic polymers (soluble); nanofibers, protein-DNA complexes; protein conjugates; erythrocytes; or virosomes. Various carrier based dosage forms comprise solid lipid nanoparticles (SLNs), polymeric nanoparticles, ceramic nanoparticles, hydrogel nanoparticles, copolymerized peptide nanoparticles, nanocrystals and nanosuspensions, nanocrystals, nanotubes and nanowires, functionalized nanocarriers, nanospheres, nanocapsules, liposomes, lipid emulsions, lipid microtubules/microcylinders, lipid microbubbles, lipospheres, lipopolyplexes, inverse lipid micelles, dendrimers, ethosomes, multicomposite ultrathin capsules, aquasomes, pharmacosomes, colloidosomes, niosomes, discomes, proniosomes, microspheres, microemulsions and polymeric micelles. Other suitable pharmaceutically acceptable excipients are *inter alia* described in Remington's Pharmaceutical Sciences, 15th Ed., Mack Publishing Co., New Jersey (1991) and Bauer et al., Pharmazeutische Technologie, 5th Ed., Govi-Verlag Frankfurt (1997).

### Additional anti-obesity agents

The pharmaceutical composition of the present invention may further comprise one or more additional agents. Preferably, said agents are therapeutically effective for treatment of obesity and/or obesity-associated conditions and, is selected from the group of energy (re-)absorption inhibitors, peripheral modulators, CNS modulators, and herbal products. Exemplary anti-obesity agents for use in accordance with the present invention have been described elsewhere herein and are also applicable for use within the pharmaceutical composition of the invention, *mutatis mutandis.*

### Route of Administration

A variety of routes are applicable for administration of the PLA inhibitor of the invention, in particular a lysophospholipid-conjugate such as UDCA-LPE, including, but not limited to, orally, topically (including intraocular, inhalative and nasal administration), rectally, transdermally, transmucosally (including sublingual and sublabial administration) subcutaneously, intravenously, intraperitoneally, and intramuscularly. Of course, any other route may readily be chosen by the person skilled in the art if desired.

Preferably, the PLA inhibitor of the invention is administered orally and/or transmucosally. Rectal applications are also envisaged and can be compounded in many forms. Liquid rectal medicine solutions are given by enema. Creams, lotions and ointments are applied externally or inserted internally using an applicator. Suppositories might be prepared by mixing medicine with a wax-like substance to form a semi-solid, bullet-shaped form that will melt after insertion into the rectum.

### Formulation

The pharmaceutical composition of the invention can be formulated in various forms, e.g. in solid, liquid, gaseous or lyophilized form and may be, inter alia, in the form of an ointment, a cream, transdermal patches, a gel, powder, a tablet, solution, an aerosol, granules, pills, suspensions, emulsions, capsules, syrups, liquids, elixirs, extracts, tincture or fluid extracts. The chosen formulation depends, i.a., on the desired route of administration.

For example, if the pharmaceutical composition of the invention is to be administered orally, and it is desired to deliver it to the intestine, the formulation should be resistant to gastric acid and enable release only once the composition has reached its desired target within the intestinal tract. Accordingly, the composition can be formulated with an enteric coating. An enteric coating as used herein refers in general to a coating that controls the location in the digestive system where a drug is released.

Suitable delivery systems for intestinal drug delivery have been reviewed by, e.g., Jain SK and Jain A. Expert Opin Drug Deliv. 2008; 5(5):483-98 and Van den Mooter G. Expert Opin Drug Deliv. 2006; 3(1):111-25. (2006). In general, suitable formulations for intestinal delivery comprise delayed release dosage forms that may be designed to provide a "burst release" or a sustained/prolonged release once they reach the target site. The person skilled in the art is aware that the proper selection of a suitable formulation approach is dependent on several factors, for example pathology of the disease, physicochemical and biopharmaceutical properties of the drug and the desired release profile of the active ingredient. Systems that are particularly suitable for intestinal delivery include prodrugs, pH-dependent delivery systems, time release/delayed systems, microbial-triggered systems, and pressure-dependent systems.

Generally, a prodrug is a composed of a drug and a carrier which are chemically coupled to each other. Upon administration, the moiety preferably maintains its integrity while passing the non-target intestinal parts until it reaches its target, such as, e.g., the colon. On reaching its final destination, the prodrug is then converted into the parent drug molecule. Site-specific drug delivery through site-specific prodrug activation can be accomplished by utilizing a specific property of the target site, e.g. a low pH or the presence of specific enzymes, including host enzymes as well as enzymes of the bacterial gut flora. E.g., when targeting drugs to the colon, a prodrug can be used that is converted into the parental drug via the action of bacterial enzymes of the gut flora, such as azoreductase, glycosidase, polysaccharidases, or cyclodextrinase. This can also be referred to as "microbial-triggered" delivery. Accordingly, prodrug approaches including azo bond prodrugs, glysoside conjugates, glucuronide conjugates, cyclodextrin conjugates, dextran conjugates and amino acid conjugates can be used to target the compound of the invention to the target site. More specifically, pectin, guar gum, inulin, locus bean gum, glucomannan, chitosan, chondroitin sulfate, hyaluronic acid, alginates, dextran, starch, amylase, cellulose, cyclodextrin, curdlan, and sclereoglucan conjugates or mixtures thereof, optionally comprising other polymers, can be used.

The rationale underlying pH-dependent intestinal delivery systems is the use of coating agents that dissolve only at a certain pH range that can be found in a specific part of the intestine. Thus, pH dependent delivery systems exploit the rising of the pH from the stomach to the large intestine. For the purpose of targeting drugs to, e.g., the colon, tablets, capsules or the like can be coated with a pH-dependent polymer that is insoluble at low pH but soluble at neutral or slightly alkaline pH. This would thus preferably release the drug in the colon. Conversely, when the target site lies in the stomach, the person skilled in the art will select a pH-dependent polymer that is insoluble at a high pH but dissolves in the acidic environment of the stomach. Exemplary pH-dependent polymers include, but are not limited to, derivatives of acrylic acid and cellulose such as polyvinyl acetate phtalathe (PVAP, e.g., Coateric ®), cellulose acetate trimellitate (CAT), hydroxypropyl methylcellulose phthalate (HPMCP), hydroxypropyl methylcellulose acetate succinate (HPMCAS), methylacrylic acid copolymer (e.g., Eudragit ®), and cellulose acetate phthalate (CAP, e.g., Aquateric®). Eudragit ® coating is preferred for the delivery of lysophospholipid-conjugates.

Time-dependent delivery systems can also be applied in order to deliver lysophospholipid-conjugates to the intestine. These systems are in general designed to resist the environment of the non-target sites of the intestine and to undergo a silent phase of predetermined time duration, after which site-specific release of the drug takes place. For example, for colon-targeted drug delivery, the silent phase is the transit time from the mouth to the terminal ileum. Examples for time-dependent delivery systems include, but are not limited to, Pulsincap® and the delayed release osmotic dosage form Oros CT®. Other time-dependent drug delivery systems comprise multiple coated oral dosage forms and enteric-coated time-release press-coated tablets (ETP tablets).

Another approach is the use of pressure-controlled drug delivery capsules (PCDC) that rely on the physiological luminal pressure in the intestine which results from peristalsis for drug release.

Other systems for site-specific drug delivery, in particular to the colon, include the CODES® technology, and the use of recombinant bacteria, e.g., *Lactobacillus sporogenes,* as a live vector system that have been genetically engineered to colonize a specific part of the intestine and produce the desired drug there.

It is to be understood that the present invention also relates to the use of a lysophospholipid-conjugate for use in the manufacture of a medicament for the treatment obesity in a subject. Also, the invention relates to the use of a lysophospholipid-conjugate for the treatment of obesity in a subject.

### Kit

It is also envisaged that the PLA inhibitor of the invention, in particular a lysophospholipid conjugate such as UDCA-LPE, is can be used as part of a kit. Accordingly, in a further aspect, the present invention also relates to a kit comprising a PLA inhibitor of the invention, in particular a lysophospholipid-conjugate such as UDCA-LPE, for use in a method of treatment of obesity.

The kit may be a kit of two or more parts, and comprises the PLA inhibitor of the invention, in particular a lysophospholipid-conjugate such as UDCA-LPE, optionally with a pharmaceutically acceptable excipient as described in the context of the pharmaceutical composition of the invention. The components of the kit may be contained in a container or vials. It is to be noted that all embodiments described in the context of the PLA inhibitor of the invention, the pharmaceutical composition comprising said PLA inhibitor and the methods of treatment can also be applied to the kit of the invention, *mutatis mutandis.*

The kit may further comprise one or more anti-obesity agents, optionally with a pharmaceutically acceptable excipient. Suitable anti-obesity agents have been described elsewhere herein and are equally applicable in the kit of the invention. It is envisaged that the agents are applied prior to, simultaneously with or after the PLA inhibitor of the invention, which is in particular a lysophospholipid such as UDCA-LPE. The present invention further encompasses the application of the kit components via different administration routes. For instance, an oral formulation of, e.g. UDCA-LPE, can be provided for use with one or more parenterally or topically administered anti-obesity agents.

Preferred anti-obesity agents are therapeutically effective for treatment of obesity and/or obesity-associated conditions and are selected from the group of energy (re-)absorption inhibitors, peripheral modulators, CNS modulators, and herbal products. Exemplary anti-obesity agents have been described elsewhere herein and are also applicable for use within the kit of the invention, *mutatis mutandis.*

### Methods

The present invention further provides a method of non-therapeutic body fat reduction in a subject, comprising administering a phospholipase A inhibitor, in particular a lysophospholipid-conjugate such as UDCA-LPE.

The person skilled in the art will acknowledge that the embodiments described herein in the context the PLA inhibitor of the invention, the pharmaceutical composition and the kit of the present invention are applicable to the method of treatment, *mutatis mutandis.* Administration of the PLA inhibitor of the invention may further be combined with psychological therapies, such as cognitive behavioral therapy, and lifestyle changes including physical exercise (e.g. 60 min per day for children, 150 min per week for adults), and/or a diet, e.g. according to the WHO recommendations, i.a. comprising
- limiting energy intake from total fats and shifting fat consumption away from saturated fats to unsaturated fats and towards the elimination of trans-fatty acids
- increasing consumption of fruits and vegetables, and legumes, whole grains and nuts
- limit the intake of free sugars
- limiting salt (sodium) consumption from all sources and ensure that salt is iodized

The method may further comprise a step of administrating one or more anti-obesity agents as described elsewhere herein.

Another aspect of the present invention is a method of treatment of obesity in a subject in need thereof, comprising administering a therapeutically effective amount of a PLA inhibitor of the invention, in particular a lysophospholipid such as UDCA-LPE, to said subject.

Additionally, the method may further comprise any of the steps described in the context of the non-therapeutic application.

It is to be understood that all embodiments, definition, etc. disclosed in the context of treatment are fully applicable to methods described herein as well.

### Food products

In another aspect, the present invention provides food product composition comprising a lysophospholipid-conjugate.

In principle, the food product composition is not limited in terms of form and type. For example, the food product composition can be in liquid, powdery, or solid form. Food product compositions of the invention substantially any overall functional foods commonly known in the art. They may further include different nutrients, vitamins, electrolytes, flavors, coloring agents, pectic acid and salts thereof, alginic acid and salts thereof, organic acid, protective colloidal thickeners, pH modifiers, stabilizers, preservatives, glycerin, alcohol, a carbonating agent used for carbonated drinks (so called 'sparkling drinks'), or the like. According to an embodiment of the present invention, the inventive composition may additionally include natural fruit juice, fruit juice beverage and/or fruit puree used for manufacturing vegetable drinks.

Examples of foodstuffs possibly containing the lysophospholipid-conjugate may include meat, sausages, bread, chocolate, candy, snacks, confections, pizza, ramen noodles, other noodles, gum, dairy products including ice cream, various soups, beverages, teas, drinks, etc.

The food product composition can be in single-dose or multi-dose form.

A better understanding of the present invention and of its advantages will be had from the following examples, offered for illustrative purposes only. The examples are not intended to limit the scope of the present invention in any way.

### Example 1: UDCA-LPE as inhibitor of hepatocellular fatty acid uptake

### Materials and Methods

### Single pass [3H]oleate extraction rates in retrogradely perfused mouse livers

Twelve-week-old female C57BL/6 mice were injected intraperitoneally with 1 ml UDCA-LPE [ChemCon, Freiburg, Germany] (Chamulitrat W. et al. Hepatology. 2009; 50: 143-154) at 23 mg/kg or phosphate-buffered saline (PBS) as a control with 5% Tween 80. After 1 h, mice were killed by an overdose of ketamine (Albrecht GmbH, Aulendorf, Germany); the abdomen was opened, and a 22G stopcock cannula was inserted into the distal inferior vena cava after its ligation close to the diaphragm. Retrograde perfusion of oxygenized 100 µM albumin (A4503, Sigma, Munich, Germany) in PBS was performed at 37°C and a flow rate of 4 ml/min, with the perfusate collected from the opened portal vein (Hoffmann MJ et al. Hepatology. 2012; 55: 1369-1378). After equilibration for 5 min, a 1 ml bolus of 200 µM [3H]oleate (Perkin Elmer, Rodgau, Germany) along with 100 µM [methyl-14 C]albumin (Biotrend, Cologne, Germany) or 100 µM [14C]sucrose (Perkin Elmer) was introduced through the stopcock and the perfusion was allowed to continue for 5 min. Outflow samples were collected every 15 s and single pass extraction rates were determined as previously described (Hoffmann MJ et al. Toxicol. Sciences. 1999; 49: 40-47).

### Fatty acid uptake and membrane binding studies

The human hepatocyte-derived tumor cell line HepG2 and hepatocytes isolated from wild-type and iPLA₂ß (Group VIA PLA₂)-null (iPLA₂ß-/-) mice (Bao S et al. J Biol Chem. 2006; 281: 20958-20973) were grown to confluence in Dulbecco's Minimal Essential Medium (Gibco Life Technologies, Darmstadt, Germany) containing 10% fetal calf serum for 16 h and preincubated with PBS, UDCA-LPE, or the iPLA₂ß inhibitors methyl arachidonyl fluorophosphonate (MAFP; BML-ST 360, Enzo Life Sciences, Lörrach, Germany) or bromoenol lactone (BEL; B 1552, Sigma) (Balsinde J and Balboa MA. Cell Signal. 2005; 17: 1052-1062). After washing with PBS, 100 µM [3H]oleate bound to albumin was added at molar ratios of 0.5:1, 1:1, 2:1, and 4:1 and cellular influx was determined at 37°C over a 1-min linear uptake period during which no efflux was detected (Stremmel W and Berk PD. Proc. Natl. Acad. Sci. U.S.A. 1986; 83: 3086-3090, Stremmel W et al. Proc. Natl. Acad. Sci. U.S.A. 1986; 83: 3584-3588). Intracellular radioactivity was determined after washing cells with 10% albumin in PBS at 4°C (Stremmel W and Berk PD., loc cit). Binding of [3H]oleate to the outer PM was determined after incubation with [3H]oleate:albumin (2:1) for 5 min at 4°C.

### Subcellular fractionation

Detergent-resistant PM (DRM-PM) and non-DRM-PM fractions were isolated and characterized as previously described (Pohl J et al. Mol Biol Cell. 2005; 16: 24-31). Subcellular membrane fractions were prepared by centrifugation at 100,000 g for 1 h to obtain homogenate pellets. Nuclear extracts were obtained by centrifugation at 500 g for 5 min. Pellets were washed and resuspended in a buffer solution containing 50 nM Tris-HCl (pH 7.4), 100 mM NaCl, and 0.1 mM EDTA (Marzluff WF et al. Meth Cell Biol. 1987; 317-331)). The protein concentration was determined by the Bradford method (Bio- Rad, Munich, Germany).

### Cellular steatohepatitis model

To induce steatosis, HepG2 cells were incubated with 400 µM oleate:albumin (4:1) for 3 h. The effect of UDCA-LPE (100 µM; 1 h) relative to PBS (control) was examined by pretreating cells before or after the 3- h fatty acid feeding period (with continuous fatty acid feeding thereafter). Fatty acid loading was examined by staining cells with Oil Red (Sigma) and by immunodetection of triglycerides (ab65336; Abcam, Cambridge, UK). In addition, the release of lactate dehydrogenase (LDH) (11644793001; Roche, Mannheim, Germany) was determined as a measure of apoptosis.

### Measurement of PLA₂ activity

PLA₂ activity (µmoles LPC/mg protein/h) was determined by incubating samples with arachidonoyl thio-PC (62240; Cayman, Tallin, Estonia) for 1 h at 25°C in a Ca2+ -free buffer (Bhat MK et al. Biochim Biophys Act. 1993; 1166: 244-250). The reaction was terminated by adding 5,5-dithio-bis-2-nitrobenzoic acid and the absorbance at 405 nm was measured.

### Measurement of LPC

LPC concentration in cytosolic supernatants was measured as previously described (Kishimoto T et al. Clin. Biochem. 2002; 35: 411-416).

### Western blotting

Immunoblotting was performed according to a standard protocol as set out in Sun Q., et al. Biol Med (Maywood). 2012; 237: 178-185. The following primary antibodies to human proteins were used: mouse a-phosphorylated c-Jun N-terminal kinase 1 (p-JNK1) (88.20; Santa Cruz Biotechnology Inc., Heidelberg, Germany) at 1:500; mouse a-CD36 (1.BB.344; Santa Cruz Biotechnology Inc.) at 1:200; rabbit a-iPLA₂ß (558-588; Abcam) at 1:500; mouse a-FABPPM (331-430; Abnova, Heidelberg, Germany) at 1:1,000; mouse a-FATP4 (1F4-1B10; Abnova) at 1:500; mouse a-caveolin-1 (C20B; Santa Cruz Biotechnology Inc.) at 1:500; mouse a-flotilin1 (F-3; Santa Cruz Biotechnology Inc.) at 1:500; and mouse a-ß-actin (AC-15; Sigma) at 1:100,000. Anti-mouse or -rabbit HRP (96910 and 107487, respectively; Dianova, Hamburg, Germany) were used at 1:10,000 dilution as secondary antibodies. To estimate the protein concentration a semi-quantitative densitometry was performed as described in Sun Q et al. (*loc cit.*).

### Gene expression analysis by semi-quantitative real time polymerase chain reaction (RT-PCR)

The following forward and reverse primers (Sigma) were used for RT-PCR: iPLA₂ß, 5'- atcctgatgaatttgagcga-3' (SEQ ID NO: 1) and 5'-caagtagaagttccttgaacg-3' (SEQ ID NO: 2); CD36, 5'-tgtaacccaggacgctgagg-3' (SEQ ID NO: 3) and 5'- gaaggttcgaagatggcacc-3' (SEQ ID NO: 4); FABPPM, 5'-gagggtcggagccagctt-3' (SEQ ID NO: 5) and 5'-gtttccccaggatggtttgg-3' (SEQ ID NO: 6); and caveolin-1, 5'-aacgttctcactcgctctctgctcgctgcg-3' (SEQ ID NO: 7) and reverse 5'-gtacacttgcttctcgctcagcac-3' (SEQ ID NO: 8). The primers for FATP4 and ß-actin (internal control) were the same as those previously used as described in Digel M et al. Am J Physiol Endocrinol Metab 2011; 301, 785-796.

### Small interfering (si)RNA knockdown

HepG2 cells were transfected with 10 nM siRNA for 16 h using Lipofectamine RNAiMAX (Invitrogen GmbH, Darmstadt, Germany) according to the manufacturer's protocol. The sense and antisense probe sequences (Sigma) were as follows: scrambled siRNA (control), gaugggaccuggccaguga (SEQ ID NO: 9) and ucacuggccaggucccauc (SEQ ID NO: 10); siRNA-iPLA₂ß (human), cccguacacccaccuau (SEQ ID NO: 11) and auagguguggguguacggg (SEQ ID NO: 12); siRNA- iPLA₂ß (mouse), cugacuacgucucaaguga (SEQ ID NO: 13) and ucacuugagacguagucag (SEQ ID NO: 14); siRNA-CD36, caaagagguccuuauacgu (SEQ ID NO: 15) and acguauaaggaccucuuug (SEQ ID NO: 16); siRNA-FABPPM, gccuuuaagagggacacca (SEQ ID NO: 17) and uggugucccucuuaaaggc (SEQ ID NO: 18); siRNA- caveolin1, cuaaacaccucaacgauga (SEQ ID NO: 19) and ucaucguugagguguuuag (SEQ ID NO: 20). The efficiency of the knockdown was confirmed by western blotting.

### Fluorescence activated cell sorting (FACS) analysis

HepG2 cell suspensions were incubated with primary antibodies against CD36 and FABPPM (TR9 monoclonal IgG1 and 1.BB.344, respectively; Santa Cruz Biotechnology Inc.) at 1:50 dilution for 1 h. After washing with PBS, cells were incubated with secondary antibodies of rabbit, anti mouse IgG-FITC (human) (SC-3589; Santa Cruz Biotechnology Inc.) diluted 1:100 in 0.5% albumin and 5 mM EDTA in PBS for 1 h, then washed and resuspended in PBS; 0.5 ml of the cell suspension was analyzed using a FACSCalibur flow cytometer (Becton Dickinson/BD Biosciences, Franklin Lake, NJ, USA). The results were quantified with CellQuestPro software (BD Biosciences) as described in Digel M et al. (loc cit.)

### Immunoprecipitation

Aliquots of isolated DRM-PM fractions (30 µg protein in 80 µl) were added to 20 µl solubilization buffer containing 0.25 mM 2-[N-Morpholino] ethanesulfonic acid (MES) buffer (pH 6.5) containing 0.15 M NaCl, 0.05% Triton X-1 00 (T 9284; Sigma), and a protein inhibitor cocktail (P 8340; Sigma) as described in Böttcher RT et al. Nat Cell Biol. 2012; 14: 584-592. Samples were incubated for 16 h at 4°C with antibodies at a 1:100 dilution; 20 µl vortexed agarose beads (SC-2003; Santa Cruz Biotechnology Inc.) were then added, and incubation resumed for 16 h at 4°C with rotation. After centrifugation for 15 min at 2,000 rpm, pellets were washed twice in MES buffer and used for western blotting.

### Immunocytochemistry

Confluent HepG2 cells fixed in 4% paraformaldehyde in PBS were labeled with antibodies against CD36 (1A/7 monoclonal IgG1; Santa Cruz Biotechnology Inc.) and iPLA₂ß (ab163258; Abcam) at 1:100 dilution for 1 h at 4°C. Cy2-conjugated secondary antibodies (015-220-003; Dianova) diluted 1:1,000 in 0.05 mM Tris-HCl (pH 7.4) were applied for 1 h at 4°C. Images were acquired using an IX50 S872 inverted fluorescence microscope (Olympus, Hamburg, Germany) with the ColorView 12 Analysis 3.00 Soft Imaging System (Olympus).

### Statistical analysis

Statistical analysis was performed using Prism software version 4.0 (GraphPad Software Inc., La Jolla, CA, USA). Differences between groups were evaluated using an unpaired two-tailed Student's *t* test. Multiple groups were compared by one-way analysis of variance with a Dunnett's post-hoc test. Data are presented as mean ± SD, and a P value < 0.05 was considered statistically significant.

### Results

### UDCA-LPE inhibits fatty acid uptake via iPLA₂ß

Oleate was selected as representative fatty acid and presented as an oleate:albumin complex to recapitulate physiological conditions. First, the general effect of UDCA-LPE on hepatic fatty acid uptake *in vivo* was determined. The single pass [ ₃ H]oleate extraction rates in retrogradely perfused mouse livers (Hoffmann MJ et al. Toxicol Sci. 2005; 49: 40-47) were analyzed with respect to the extracellular marker [ ₁₄ C]albumin or [ ₁₄ C]sucrose (Goresky CA et al. Circ Res. 1994; 74: 1015-1026). The cumulative roportion of the [ ₃ H]oleate extracted from 200 µM [ ₃ H]oleate:albumin (2:1) decreased from 0.41 ± 0.02 in controls to 0.30 ± 0.03 in mice pretreated with UDCA-LPE, corresponding to a 34 % inhibition of fatty acid influx (P < 0.05). To evaluate the underlying mechanism of fatty acid influx inhibition in more detail, the human hepatoma cell line HepG2 was treated with UDCA-LPE. Fatty acid influx decreased until reaching a minimum after 60 min but the original rate was restored 6 h after UDCA-LPE withdrawal (Fig. 1A). UDCA-LPE is transported into the cell by temperature-sensitive bile salt carriers (Na+ taurocholate cotransporting polypeptide in hepatocytes and organic anion-transporting polypeptide 1 in HepG2 cells); thus, bile acids such as UDCA compete with UDCA-LPE for uptake by HepG2 cells, with a half-maximal inhibitory concentration (IC50) of 20 µM (Chamulitrat W et al., Hepatology. 2009; 50: 143-154). HepG2 cells were pretreated with UDCA-LPE and then incubated with [ ₃ H]oleate:albumin at 4°C and 37°C to measure binding to the outer PM and influx, respectively; these were only inhibited in cells preconditioned at 37°C (Fig. 1 B). The influx of [ ₃ H]oleate was non-competitively inhibited by UDCA-LPE with a Vmax of 28.43 nmol/mg protein/min (confidence interval, CI: 23.47-33.39) compared to 61.80 nmol/mg protein/min (CI: 48.98-74.63) in controls (P < 0.001); no significant difference in Km (250.5 nM, CI: 105.3-395.7 vs. 416.3 nM, CI: 139.2-693.3) was observed. To test the hypothesis that PLA₂ is inhibited by UDCA-LPE, HepG2 cells were pretreated with the iPLA₂ß inhibitor MAFP (Pathil A et al. J. Hepatology. 2010; 54: 674-684); a similar non-competitive inhibition of fatty acid influx was observed, with Vmax decreasing to 26.38 nmol/mg protein/min (CI: 21.15-31.62) (P < 0.001), while there was no change in Km (303.3 nM, CI: 106.6-500.0) (Fig. 1C). The inhibition of fatty acid influx by UDCA-LPE (IC50: 47 µM) was correlated in a dose-dependent manner with the suppression of PLA₂ activity; moreover, the inhibition of iPLA₂ß by treatment with MAFP and BEL induced a dose-dependent decrease in fatty acid influx (IC50: 56 and 52 µM, respectively) (Fig. 2A). In all instances, the suppression of PLA₂ activity was observed exclusively in the DRM-PM, representing structured lipid membrane platforms constituting lipid rafts, which mediate cellular communication (Stremmel W et al. Lipids. 2001; 36, 981-989) (Fig. 2B). Thus, DRM-PM-localized iPLA₂ß is the substrate for UDCA-LPE, and can interact with a fatty acid transport system also present in this fraction.

### Fatty acid uptake is mediated by a protein complex of CD36, FABPPM, and caveolin1 in conjunction with iPLA₂ß in the DRM-PM

Three proteins involved in fatty acid transport have been proposed to be associated with the plasma membrane: FABPPM and CD36, which extend to the outer surface, and caveolin1, located at the inner bilayer (Stremmel W et al. Lipids. 2001; *loc cit.*) As a peripheral membrane protein with no known fatty acid-binding capacity, iPLA₂ß likely interacts with the members of this complex at the inner membrane surface. To investigate the composition and localization of the fatty acid transport system within the DRM-PM, membranes were isolated from HepG2 cells (Pohl J et al. Mol. Biol. Cell. 2005; 16: 24-31) and immunoprecipitated with antibodies against either iPLA₂ß, CD36, FABPPM, or caveolin1, and precipitates were probed for each of the other presumed complex subunits as well as flotilin1, a DRM-PM marker protein. FABPPM, CD36, caveolin1, and iPLA₂ß were detected in all of the precipitates, suggesting that they interact within DRM-PM (Fig. 3A). Consistent with this supposition, immunofluorescence analysis revealed the colocalization of iPLA₂ß and CD36 at the plasma membrane (Fig. 3B). A functional analysis of the fatty acid uptake complex was performed by knocking down each constituent in HepG2 cells by siRNA treatment (see Sun Q et al. Biol. Med. (Maywood). 2012; *loc. cit.*)*.* Knockdown of any one of the subunits resulted in the depletion of all subunits in the isolated DRM-PM fraction of pretreated HepG2 cells (Fig. 4A). Correspondingly, fatty acid influx was reduced by about 60% in all knockdown conditions, with a diminished sensitivity to UDCA-LPE (Fig. 4A). The disruption of lipid rafts by application of methyl-beta-cyclodextrin (MßCD) reduced fatty acid influx by an additional 60% (Fig. 4A), but not influx of [ ₃ H]glucose (135.70 ± 14.7 vs. 129.71 ± 13.8 nmol/mg protein/min at 10 mM glucose). These results confirm that fatty acid uptake activity is associated with the DRM-PM. Moreover, when protonation of fatty acids was prevented by including Na-gluconate in the apical medium (Digel), the passive translocation component was inhibited to negligible levels. To determine whether the above observations hold *in vivo,* primary hepatocytes isolated from wild- type and iPLA₂ß-/ - mice (Bao S et al. J Biol Chem. 2006; 281: 20958-20973) were cultured for 16 h and siRNA knockdown of the fatty uptake complex subunits was performed in the absence or presence of UDCA-LPE. Fatty acid influx was inhibited by UDCA-LPE in wild-type but not iPLA₂ß-/ hepatocytes (Fig. 4B), confirming that the observations made in HepG2 cells can be generalized to all liver cells. Moreover, pretreatment of wild-type hepatocytes with siRNA against fatty acid uptake complex components reduced influx and rendered cells non-responsive to UDCA-LPE. Fatty acid influx was also lower in hepatocytes derived from iPLA₂ß-/ - mice compared to those from the wild-type, with no sensitivity to UDCA-LPE and siRNA pretreatment.

### iPLA₂ß is a core component of the fatty acid uptake complex and acts as a metabolic checkpoint

To investigate the dynamics of the complex, the localization of the constituent proteins was assessed over time and as a function of UDCA-LPE concentration after treatment of HepG2 cells and subsequent isolation of the DRM-PM (Fig. 5A, B). Starting at 50 µM UDCA-LPE and 30 min of incubation, the fatty acid uptake complex began to disassemble, with iPLA₂ß and CD36 the first to dissociate. Comparable results were obtained upon treatment with MAFP and BEL. The fact that it was possible to isolate the DRM-PM from HepG2 cells after the pretreatment and the persistence of flotilin1 within this fraction indicate that the disintegration of the complex was not due to a detergent effect of UDCA-LPE. After 30 min, most of the iPLA₂ß, CD36, FABPPM, and caveolin1 had translocated from the DRM-PM to the non-DRM-PM fraction and then to subcellular membranes, where protein degradation is initiated (Fig. 5C). The displacement of the outer membrane fatty acid-binding proteins CD36 and FABPPM from the DRM-PM was time-dependent, as demonstrated by western blotting and FACS analysis (Fig. 5D). The lysosomal degradation pathway was presumed as the likely mechanism responsible for the degradation of the fatty acid uptake complex, because pretreatment with the lysosomal blocking agent bafilomycin (50 nM, 16 h) (Böttcher RT et al. Nat Cell Biol. 2012; loc. cit) resulted in an increase in all four DRM-PM proteins: iPLA₂ß by 51.56 ± 9.85%, CD36 by 40.85 ± 10.56%, FABPPM by 25.99 ± 3.35%, and caveolin1 by 60.76 ± 7.03%, as determined by semi-quantitative densitometry. Fatty acid influx was also increased by this treatment, from 26.95 ± 2.4 to 33.33 ± 5.1 nmol/mg protein/min (P < 0.01). In contrast, preincubation with the proteasomal inhibitor MG132 (5 µM, 16 h) (Böttcher RT et al. *Nat Cell Biol.* 2012; loc. cit) had no effect on uptake.

### Changes in fatty acid metabolism are induced by UDCA-LPE

To determine whether UDCA-LPE modulates the synthesis of fatty acid uptake complex components, transcript levels of each subunit were measured by RT-PCR as a function of UDCA-LPE exposure time. After 1 h of treatment, transcript levels of all components were simultaneously reduced to < 10% of initial values, and recovered concurrently after UDCA-LPE withdrawal. In contrast, the mRNA level of FATP4 as negative control remained unaltered (Fig. 6). Given these results, UDCA-LPE was hypothesized as creating a metabolic milieu that can sense a change in the mRNA levels of the membrane fatty acid uptake complex components via the UDCA-LPE target iPLA₂ß, which enzymatically alters the cellular PC/LPC ratio, thereby releasing LPC into the cytosol to regulate signal transduction (Fang XJ et al. Biol Chem. 1997; 272: 13683-13689, Kakisaka K et al. Am. J. Physiol. Gastrointest. Liver Physiol. 2012; 302: 677-684). Suppression of PLA₂ activity by UDCA-LPE decreased cytosolic LPC from 8.0 ± 1.3 to 3.5 ± 0.2 µmol/mg protein and concomitantly reduced p-JNK1 levels (Fig. 6). The p-JNK1 activation of its downstream transcriptional targets, *e.g.,* activator protein 1, regulates fatty acid metabolism and hepatic lipoapoptosis (Fang XJ et al. *loc. cit.,* Kakisaka K et al. *loc. cit.,* Seki E et al., Gastroenterology 2012; 143: 307-320). Thus, the rapid and efficient UDCA-LPE-mediated downregulation of fatty acid transporters via low JNK1 phosphorylation completes the circle of events (Fig. 6). The suppression of cytosolic LPC was also observed by treatment of cells with MAFP and BEL (IC50 MAFP and BEL = 62 and 43 µM, respectively, compared to 47 µM for UDCA-LPE) (Fig. 7). The decrease in p-JNK1 began at a threshold LPC concentration of = 5 µmol/mg protein, which was achieved by pretreatment with > 50 µM UDCA-LPE for > 30 min. To test the hypothesis that low cytosolic LPC suppresses JNK1 phosphorylation, which in turn inhibits transcription of fatty acid uptake complex subunits, delipidated cytosol (Cham BE and Knowles BR. J. Lip. Res. 1976; 17: 176-181) of HepG2 cells not previously exposed to UDCA-LPE were incubated with various concentrations of LPC, and p-JNK1 levels were measured 1 h later. *In vitro* transcription was then performed with native HepG2 nuclear extracts (Marzluff WF et al. *loc cit.*) exposed to the LPC-conditioned cytosolic samples. At LPC concentrations = 5 µmol/mg protein, the transcripts of all complex subunits were downregulated (Fig. 7).

### UDCA-LPE suppresses steatohepatitis in an in vitro model

To recapitulate a metabolic milieu similar to that of NASH, HepG2 cells were incubated for 3 h with a high concentration of free fatty acid to induce steatosis and cell injury. UDCA-LPE prevented or reversed steatosis as well as fatty acid-induced inflammation (LDH release) when added before or after the induction of steatosis, respectively (Fig. 8). It acted through PLA₂ suppression resulting in low LPC and consequent reduction in p-JNK1 levels. In addition, UDCA-LPE disintegrated the operative unit of fatty acid transport within the DRM-PM by removal of iPLA₂ß from the complex. Thus, steatosis is prevented by the dual action of UDCA-LPE, which disables the mechanism of fatty acid uptake that leads to cellular starvation even in the presence of excess fatty acids.

### Discussion

Using UDCA-LPE, a plasma membrane fatty acid uptake complex residing within the DRM-PM was found that contained fatty acid-binding and translocation moieties as well as the key regulatory component iPLA₂ß. PLA₂ is accessible to various cellular metabolites (Balsinde J and Balboa MA. Cell Signal. 2005; 17: 1052-1062) and regulates fatty acid influx according to cellular demands. The present findings elucidated a previously unidentified mechanism of fatty acid uptake in HepG2 cells, which were used in these experiments in order to obtain robust and reproducible data. The results were confirmed in a key experiment using mouse hepatocytes from wild-type and iPLA₂ß-/ - mice (Bao et al., *loc cit.*)*.*

### Identification of a fatty acid uptake complex within the DRM-PM

This is the first demonstration of the localization of known membrane fatty acid-binding proteins CD36, FABPPM, and caveolin1 within a common PM platform, and their interaction with membrane-localized iPLA₂ß to form a heterotetrameric complex. These interactions were confirmed by immunoprecipitation: when a subunit-specific antibody was used to precipitate any member of the complex from isolated native DRM-PM, the other three members were also detected (Fig. 3). It is worth noting that the raft marker protein flotilin1 always co-precipitated with the complex. Likewise, when siRNA was used to knock down the expression of any member, the expression of the remaining members, but not of flotilin1, was simultaneously downregulated (Fig. 4). Moreover, the influx of fatty acids was concomitantly inhibited, underscoring the function of this complex in fatty acid uptake. The outer proteins of the DRM-PM platform are the fatty acid-binding proteins CD36 and FABPPM (described here for the first time as a transient DRM-PM protein), which can capture circulating fatty acids by virtue of their high binding affinities. The protonization of fatty acids facilitates their flip-flopping across the lipid bilayer (Stremmel W et al. Lipids. 2001; *loc cit.*) to the inner surface of the PM, where they are bound by caveolin1 and are presented to the cytosolic acceptor protein FABPC or else directed to the metabolic pathway via acyl-CoA activation.

### Contribution of iPLA₂ß to the fatty acid uptake complex structure

The fact that the newly designed phospholipid-bile acid conjugate UDCA-LPE acts as an iPLA₂ß inhibitor that acts exclusively at the DRM-PM, similar to the known inhibitors MAFP and BEL, was unexpected. Although iPLA₂ß is not directly involved in fatty acid binding to the membrane, it is an integral component that maintains the structure of the fatty acid uptake complex within the DRM-PM. Thus, iPLA₂ß inhibition by UDCA-LPE treatment resulted in the disintegration of the heterotetramer (Fig. 5). CD36 was the first to dissociate, and appeared to be the primary anchor for iPLA₂ß; this was followed by FABPPM and caveolin1. The movement of the four complex constituents out of the DRM-PM platform resulted in an intermittent, non-functional, labile assembly within the non-DRM-PM. With this transition, the facilitated fatty acid uptake was replaced by passive DRM-PM-dependent fatty acid transport, which exhibited a reduced sensitivity to UDCA-LPE (Fig. 4A). Indeed, when cells devoid of fatty acid transporters were exposed to MßCD (which destroyed DRMs), the fatty acid influx rate was reduced by an additional 60% (Fig. 4A) (Stremmel, W. J. Biol. Chem. 1987; 262: 6284-6289).

### Role of iPLA₂ß in fatty acid metabolism

iPLA₂ß controls fatty acid influx by cleaving PC to LPC, which accumulates in the cytosol. Above the crucial concentration of 5 µmol/mg protein, LPC activates JNK1, a key regulator of metabolism, inflammation, apoptosis, and regeneration (Fang XJ et al. *loc. cit.,* Kakisaka K et al. *loc. cit.,* Seki E et al. *loc. cit.*)*.* The present data indicate that fatty acid influx is enhanced by the increased synthesis of the four proteins of the fatty acid uptake complex, which promotes steatosis; the LPC/p-JNK1-dependent induction of lipoapoptosis represents the other classical feature of NASH. Accordingly, *in vivo* deletion of iPLA₂ß in a mouse model is predicted to induce a hypolipidemic state in the liver. Human studies have shown that a mutation in *PNPLA3,* a member of the iPLA₂ family, conferred protection against NASH, as demonstrated by reduced very low-density lipoprotein and triglyceride levels, and decreased cholesterol secretion into the blood (Krarup, NT et al. PlosOne. 2012; 7: e40376). The homolog of the here examined Group VIA PLA₂ iPLA₂ß is encoded by *PNPLA9.* It is therefore of great interest to determine whether mutations in this gene can also confer protection against NASH.

### Strategies for therapeutic intervention via iPLA₂ß inhibition

iPLA₂ß may serve as an ideal therapeutic target for NASH treatment because its inhibition by UDCA-LPE can reverse cellular fat accumulation and inflammatory response as shown in our previous NASH mouse models (Chamulitrat W et al. Hepatology 2009; 50: 143-154) and confirmed here in HepG2 cells exposed to a similar cellular milieu. Intact UDCA-LPE could compete as a modified phospholipid with PC for the acceptor site of iPLA₂ß: its lower dissociation rates could inhibit the enzymatic activity of iPLA₂ß, suppress LPC generation, and consequently reduce JNK1 phosphorylation, leading to the inhibition of fatty acid transporter biosynthesis. The competition of UDCA-LPE with PC may also induce the displacement of iPLA₂ß from the DRM-PM-localized fatty acid uptake complex, triggering its disintegration. The advantage of this conjugate compared to other iPLA₂ß inhibitors is its non-toxicity, because its breakdown products are degraded by the cellular metabolic machinery (Chamulitrat W et al. Hepatology 2009; 50: *loc. cit.,* Chamulitrat W et al. Front. Physiol. 2012; 3: 24). Thus, the development of an ingestible form of UDCA-LPE, and a validation of its clinical efficacy, would offer a viable, potential treatment strategy for NASH.

### Example 2: UDCA-LPE as inhibitor of fatty acid uptake in mucosal cells and fatty acid absorption

### Materials and Methods

For the following experiments, CaCo2 cells were used. Fatty acid uptake and membrane binding studies were conducted with CaCo2 cells cultured for 3-30 days cultured.

All other experiments were assessed with CaCo2 cells as described in Ex. 1, *mutatis mutandis.*

### Results

Preincubation of the intestinal mucosa derived cell line CaCo2 with UDCA-LPE revealed non-competitive inhibition of fatty acid influx kinetics which was compatible with the known PLA₂ inhibitor MAFP ( ) and comparable to the data obtained in HepG2 cells.

To evaluate whether the interaction of UDCA-LPE occurs at the outside or inside surface of the plasma membrane, CaCo2 cells were incubated with UDCA-LPE at 37°C which allows cellular uptake via bile salt carrier proteins, and at 4°C which allows just binding to the outer plasma membranes. After warming-up of the tissue cultures to 37°C influx was only inhibited in 37°C preconditioned cells. The same was true for fatty acid binding to plasma membranes when examined at 4°C. This indicates that UDCA-LPE must first be internalized before its action can be initiated by interacting with a phospholipase A2 at the inner side of the plasma membrane. When increasing concentrations of UDCA-LPE or the PLA inhibitors MAFP or BEL were analyzed in regard to their PLA₂ inhibition and oleate uptake capacities, the corresponding values overlapped. The PLA₂ which was inhibited by UDCA-LPE, MAFP and BEL resided in the detergent resistant membrane (DRM) domain of the plasma membranes. It was assumed from the studies in the hepatocyte derived tumor cell line HepG2 that it is most likely the calcium independent membrane associated iPLA₂ß.

For molecular characterization of the fatty acid uptake complex, the DRM domains of the plasma membrane were isolated from native CaCo2 cells and immunoprecipitated with antibodies to the known membrane fatty acid binding proteins FABPPM, CD36 and caveolin1 as well as iPLA₂ß. The DRM marker protein flotilin1 served as control. It was shown that each immunoprecipitate contained all of the other members, indicative for a heterotetrameric protein complex with the DRM domain of the plasma membrane. Immunofluorescence analysis confirmed for CD36 and PLA₂ costaining at the level of the plasma membrane.

For functional analysis CaCo2 cells were pretreated for 16 h with siRNA's to each of the four members of the uptake complex and the impact of additional UDCA-LPE (100 µM, 1 h) on influx of fatty acids was determined. While under control conditions (scrambled siRNA) UDCA-LPE inhibited influx by > 50%, in all other knock-down settings uptake was already markedly reduced and UDCA-LPE did not further suppress fatty acid influx. Accordingly, the members of the uptake complex had all disappeared from DRM domains of the plasma membrane. Furthermore, analysis of the disintegration process of the complex revealed a minimal concentration of 50 µM UDCA-LPE and exposure time of 30 min. The members of the uptake complex then subsequently moved from DRM to non-DRM plasma membrane domains and then to subcellular membranes, mostly lysosomes where degradation of these proteins occurs. Next the metabolic effect induced by the UDCA-LPE pretreatment of CaCo2 cells was evaluated. As a function of time of exposure and withdrawal of the drug, phospholipase activity and cytosolic LPC concentration dropped and returned back to normal, respectively. This corresponded to the concentration of JNK1p which results in synthesis of the four members of the fatty acid uptake complex as quantified by real-time PCR. Accordingly protein levels of those four proteins vary with exposure/withdrawal of UDCA-LPE.

The above experiments confirm the data obtained in HepG2 cells and establish the presence of a four-protein fatty acid uptake complex within the DRM-domain of CaCo2 plasma membranes. However, in vivo mucosal cells are polarized with the apical membrane serving absorption of monomeric luminal substrates, including fatty acids and cholesterol, whereas the basal plasma membrane releases lipoprotein incorporated substrates. Thus, the fatty acid transport complex should predominantly be localized at the apical side. Accordingly, CaCo2 cells were cultured for 21 days which allows full apical/basolateral polarization revealing a transepithelial resistance (TER) >450Ω.

When the time course of cellular fatty acid influx from apical was evaluated, it was evident that apical influx is linear and significantly inhibited by UDCA-LPE pretreatment at the apical site. The inhibition was not only recorded for fatty acids (saturated and non-saturated), but also for lysosphospholipids and, importantly, for cholesterol. For those experiments the Na+-salt of UDCA-LPE was superior than acidic UDCA-LPE because the cellular influx of Na+ UDCA-LPE was more efficient.

To determine the effect of Na+ UDCA-LPE in vivo on fatty acid absorption, mice received by oral gavage 100 µl 1 mM UDCA-LPE in 5 % Tween 80 vs. 100 µl 5 % Tween 80 alone, 1 h before 100 µl of 100 mM [³H]oleateaaurocholate (1:1) (500.000 cpm) were provided also by oral gavage. After 1 h the animals were sacrified and samples of blood and representative organs were analyzed in regard to recovered [³H]oleate. The diminished absorption due to Na+ UDCA-LPE prepreatment was robust with less than 7 % recovery in blood compared to controls. A significant amount of oleate was retained in the mucus compartment, from where normally fatty acid absorption takes place. The UDCA-LPE inhibition of fatty acid absorption was only observed for the Na+-salt and time dependent. It indicates that Na+ UDCA-LPE is a potent drug to inhibit fatty acid absorption from intestine acting on the apical side of mucosal cells. Its mode of action after absorption through bile acid carriers relates to disintegration of the membrane fatty acid uptake complex and inhibition of the protein synthesis of all four members constituting this transport system.

It was further hypothesized that UDCA-LPE can interfere via iPLA₂ß inhibition with the membrane location of CD36 in tongue epithelium and duodenal mucosa (Degrace-Passily & Besmand, 2012). Here CD36 as part of the fatty acid recognition complex serves as fatty acid receptor and signals from tongue to brain for appetite regulation and from duodenum to pancreas for endocrine and exporine hormonal/enzyme secretion. When CD36 is displaced, loss of appetite and malabsorption is expected but also improved insulin sensitivity. As initial proof of principle experiment we determined the CD36 protein content of tongue tissue in control and iPLA₂ß (-/-) mice.

It was indeed shown that lack of iPLA₂ß results in fading of CD36 and FABPPM. Indeed these mice reveal a hypolipidemic phenotype.

## Claims

1. A phospholipase A inhibitor for use in a method of therapeutically reducing fat absorption in a subject.

2. The phospholipase A inhibitor for the use of claim 1, which is for the therapeutic reduction of body fat in a subject.

3. The phospholipase A inhibitor for the use of claim 1 or 2, wherein the method is for treatment and/or prevention of obesity in a subject.

4. The phospholipase A inhibitor for the use of claim 1, 2 or 3, wherein said inhibitor is a lysophospholipid-conjugate wherein
the lysophospholipid could be chemically coupled to a bile acid and
wherein the bile acid could be ursodeoxycholate or deoxycholate.

5. The phospholipase A inhibitor for the use of claim 4, wherein the lysophospholipid is lysophosphatidylethanolamine or lysophosphatidylcholine.

6. The phospholipase A inhibitor for the use of any of claims 1 to 5, wherein said inhibitor is orally administered.

7. The phospholipase A inhibitor for the use of any of claims 1 to 6, wherein the method further comprises administering one or more anti-obesity agents prior to, simultaneously with, or after the phospholipase A inhibitor,
wherein the one or more anti-obesity agents could be selected from energy (re-) absorption inhibitors, peripheral modulators, CNS modulators, and herbal products.

8. The phospholipase A inhibitor for the use of any of claims 1 to 7, the method further comprising subjecting the subject to a psychological training, a diet and/or regular exercise.

9. A phospholipase A inhibitor for use in non-therapeutic body fat reduction and/or control of a subject.

10. A method of non-therapeutic body fat reduction and/or control in a subject, comprising administering a phospholipase A inhibitor.

11. The method of claim 10, further comprising administering one or more anti-obesity agents prior to, simultaneously with, or after the phospholipase A inhibitor

12. A pharmaceutical composition for use in treatment of obesity in a subject, comprising a lysophospholipid-conjugate and a pharmaceutically acceptable excipient.

13. The pharmaceutical composition of claim 12, further comprising one or more anti-obesity agents.

14. A lysophospholipid-conjugate for use in the manufacture of a medicament for the treatment obesity in a subject.

15. Use of a lysophospholipid-conjugate for the treatment of obesity in a subject.

16. A kit for use in the treatment of obesity in a subject, comprising a lysophospholipid-conjugate and one ore more anti-obesity agents.

17. A food product composition comprising a lysophopspholipid-conjugate.
